# EUROPEAN PATENT SPECIFICATION

(11) **EP 4 022 287 B1**
(45) Date of publication and mention of the grant of the patent: **30.07.2025**
(21) Application number: 20771987.3
(22) Date of filing: 26.08.2020
(51) Int. Cl.: G01N 21/35, G01N 21/65

(54) **SYSTEMS AND METHODS FOR ASSESSING SPECIMEN FIXATION DURATION AND QUALITY USING VIBRATIONAL SPECTROSCOPY**
SYSTEME UND VERFAHREN ZUR BEURTEILUNG DER FIXIERDAUER UND DER QUALITÄT VON PROBEN MITTELS VIBRATIONSSPEKTROSKOPIE
SYSTÈMES ET PROCÉDÉS D'ÉVALUATION DE DURÉE ET DE QUALITÉ DE FIXATION D'ÉCHANTILLON PAR SPECTROSCOPIE VIBRATIONNELLE

(30) Priority: 28.08.2019 US 201962892678 P
(43) Date of publication of application: 06.07.2022
(73) Proprietor: Ventana Medical Systems, Inc., Tucson, Arizona 85755 (US)
(72) Inventor: BAUER, Daniel, Tucson, Arizona 85755 (US); CHAFIN, David, Tucson, Arizona 85755 (US)
(74) Representative: Altmann Stößel Dick Patentanwälte PartG mbB
(86) International application number: PCT/EP2020/073787
(87) International publication number: WO 2021/037875

(56) References cited:
- WO-A1-2017/072320

## Description

### CROSS-REFERENCE TO RELATED APPLICATIONS

The present application claims the benefit of the filing date of United States Patent Application No. 62/892,678 filed on August 28, 2019.

### BACKGROUND OF THE DISCLOSURE

Immunohistochemical (IHC) slide staining can be utilized to identify proteins in cells of a tissue section and hence is widely used in the study of different types of cells, such as cancerous cells and immune cells in biological tissue. Thus, IHC staining may be used in research to understand the distribution and localization of the differentially expressed biomarkers of immune cells (such as T-cells or B-cells) in a cancerous tissue for an immune response study. For example, tumors often contain infiltrates of immune cells, which may prevent the development of tumors or favor the outgrowth of tumors.

In-situ hybridization (ISH) can be used to look for the presence of a genetic abnormality or condition such as amplification of cancer-causing genes specifically in cells that, when viewed under a microscope, morphologically appear to be malignant. In situ hybridization (ISH) employs labeled DNA or RNA probe molecules that are anti-sense to a target gene sequence or transcript to detect or localize targeted nucleic acid target genes within a cell or tissue sample. ISH is performed by exposing a cell or tissue sample immobilized on a glass slide to a labeled nucleic acid probe which is capable of specifically hybridizing to a given target gene in the cell or tissue sample. Several target genes can be simultaneously analyzed by exposing a cell or tissue sample to a plurality of nucleic acid probes that have been labeled with a plurality of different nucleic acid tags. By utilizing labels having different emission wavelengths, simultaneous multicolored analysis may be performed in a single step on a single target cell or tissue sample.

Thin tissue sections are used in histology in order to obtain representative information about a tissue sample. The quality of the thin section should meet a number of characteristics in order to be properly representative of the overall tissue region where excision of the sample was performed. Although guidelines can vary according to tissue type and use, the size of the thin section generally should not be less than 2 µm. Typically, tissue sections are prepared in the range between 2 and 5 µm and should not vary in thickness by more than 50% over the lateral extent of the thin section in order to allow for appropriate further processing. Further factors that affect tissue section quality may include proper sample moisture and the temperature maintained during the sectioning process.

Formalin has been used by the histology field for over half a century. When used at room temperature, formalin diffuses into a tissue section and crosslinks proteins and nucleic acids, thereby halting metabolism, preserving biomolecules, and readying the tissue for paraffin wax infiltration. In practice, formalin fixation primarily occurs at room temperature or higher. Some groups perform fixation at slightly elevated temperatures, presumably to increase the cross-linking rate. Just as heat increases cross-linking rate, cold formalin significantly decreases cross-linking rate. For this reason, histologists typically perform tissue fixation at room temperature or higher. Some groups have used cold formaldehyde, but only in specialized situations and not for fixing tissues. For instance, groups use cold formalin to examine lipid droplets or other special situations.

Several effects are observed in tissues that are either under exposed or over exposed to formalin. If a tissue sample is not treated with formalin for a sufficiently long period of time, tissue morphology is typically very poor when the tissues are subjected to standard tissue processing. For example, in inadequately fixed tissue, subsequent exposure to ethanol shrinks the cellular structures and condenses nuclei since the tissues will not have the chance to form a proper crosslinked lattice. When under fixed tissue is stained, such as with hematoxylin and eosin (H&E), many white spaces are observed in between the cells and tissue structures, condensed nuclei and loss of cytoplasm, and samples appear pink and unbalanced with the hematoxylin stain. Tissues that have been exposed to formalin too long typically do not work well for subsequent immunohistochemical processes, presumably because of nucleic acid and/or protein denaturation and degradation. As a result, the optimal antigen retrieval conditions for these tissues do not work properly and therefore the tissue samples appear to be under stained.

Proper medical diagnosis and patient safety require properly fixing the tissue samples prior to staining. Accordingly, guidelines have been established by oncologists and pathologists for proper fixation of tissue samples. For example, according to the American Society of Clinical Oncology (ASCO), the current guideline for fixation duration in neutral buffered formalin solution for HER2 immunohistochemistry analysis is at least 6 hours, preferably more, and up to 72 hours. It would be advantageous to develop a process for rapidly fixing tissue samples both to better preserve biological molecules and tissue morphology before significant degradation occurs, and to provide accurate test results to medical professionals and patients as quickly as possible.

### BRIEF SUMMARY OF THE DISCLOSURE

Variations in the fixation of a biological specimen, e.g. a tissue sample, may impact downstream labeling and/or staining processes, which may result in inconclusive results and/or a misdiagnosis. Advantageously, the disclosed systems and methods provide for a determination of a fixation status of a tissue sample, thereby facilitating a determination of the quality of a fixed biological specimen. In some embodiments, the predicted fixation status is a quantitative determination of a fixation duration.

Applicants have surprisingly discovered that the systems and methods of the present disclosure provide for an accurate prediction of the fixation status of a test biological specimen that has been subjected to a fixation process for an unknown amount of time. Applicant further submits that the trained fixation estimation engines of the present disclosure allow for the estimation of fixation status to be made with high accuracy, even when the trained fixation estimation engine is applied to different tissue types or tissue types in which the fixation estimation engine was not previously trained. Moreover, Applicant submits that the presently disclosed systems and methods provide for a quantitative estimation of a fixation duration of a test biological specimen subjected to one or more fixation processes for unknown amounts of time, not just an estimate of whether the unknown test biological specimen was qualitatively "fixed" or "unfixed." These and other features are described further herein and illustrated in the examples and figures appended hereto.

A first aspect of the present disclosure is a system for quantitatively determining an estimated fixation duration of an at least partially fixed test biological specimen, the system comprising: (i) one or more processors, and (ii) one or more memories coupled to the one or more processors, wherein the one or more memories are a non-transitory computer-readable medium according to any one of the non-transitory computer-readable medium described above or described in further detail below, the one or more memories storing computer-executable instructions that, when executed by the one or more processors, cause the system to perform operations comprising:
a. obtaining test spectral data from the at least partially fixed test biological specimen, wherein the obtained test spectral data comprises vibrational spectral data derived from at least a portion of the biological specimen, wherein the obtained test spectral data comprises an averaged vibrational spectrum derived from a plurality of normalized and corrected vibrational spectra, wherein the plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region;
b. deriving fixation features from the obtained test spectral data using a trained fixation estimation engine; and
c. quantitatively determining the estimated fixation duration of the at least partially fixed biological specimen based on the derived fixation features, wherein an amount of time the at least partially fixed test biological specimen has been subjected to a fixation process is predicted.
In some embodiments, the vibrational spectral data includes mid-infrared (mid-IR) spectral data. In some embodiments, the vibrational spectral data includes Raman spectral data. In some embodiments, the system further includes operations for estimating a fixation quality using the trained fixation estimation engine.

In some embodiments, the fixation estimation engine is trained using training spectral data sets acquired from a plurality of differentially fixed training biological specimens. In some embodiments, the fixation estimation engine is trained using one or more training spectral data sets, wherein each training spectral data set includes a plurality of training vibrational spectra derived from a plurality of differentially fixed training tissue samples, and wherein each training vibrational spectrum includes class labels of a known fixation duration. In some embodiments, the class labels of known fixation duration are verified through functional IHC testing. In some embodiments, the class labels further include fixation quality annotations.

In some embodiments, each training spectral data set is derived by: (i) obtaining a training biological specimen; (ii) dividing the obtained training biological specimen into a plurality of training tissue samples; and (iii) fixing each training tissue sample of the plurality of training tissue samples for a different pre-determined amount of time. In some embodiments, the different pre-determined amount of time range from between about 0 hours to about 24 hours. In some embodiments, the different pre-determined amount of time range from between about 0 hours to about 12 hours.

The test spectral data includes an averaged vibrational spectrum derived from a plurality of normalized and corrected vibrational spectra. The plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region. In some embodiments, the acquired vibrational spectrum from each individual region is corrected by: (i) compensating each acquired vibrational spectrum for atmospheric effects to provide an atmospheric corrected vibrational spectrum; and (ii) compensating the atmospheric corrected vibrational spectrum for scattering. In some embodiments, wherein the regions are selected randomly.

In some embodiments, the trained fixation status estimation engine includes a machine learning algorithm based on dimensionality reduction. In some embodiments, dimensionality reduction includes a projection onto latent structure regression model. In some embodiments, the dimensionality reduction includes a principal component analysis and optionally discriminant analysis. In some embodiments, the trained fixation status estimation engine includes a neural network.

In some embodiments, the system further includes operations for assessing whether the biological specimen includes a fixation state suitable for labeling with one or more specific binding entities. In some embodiments, the system further includes operations for identifying at least one spectral band within the test data which is positively associated with biological specimen fixation.

In some embodiments, the obtained test spectral data comprises vibrational spectral information for wavelengths ranging from between about 3200 to about 3400 cm⁻¹. In some embodiments, the obtained test spectral data comprises vibrational spectral information for wavelengths ranging from between about 2800 to about 2900 cm⁻¹. In some embodiments, the obtained test spectral data comprises vibrational spectral information for wavelengths ranging from between about 1020 to about 1100 cm⁻¹. In some embodiments, the obtained test spectral data comprises vibrational spectral information for wavelengths ranging from between about 1520 to about 1580 cm⁻¹.

A second aspect of the present disclosure is a non-transitory computer-readable medium storing instructions for determining an estimated fixation duration of an at least partially fixed test biological specimen comprising:
(a) obtaining test spectral data from the test biological specimen, wherein the obtained test spectral data comprises vibrational spectral data derived from at least a portion of the biological specimen, wherein the obtained test spectral data comprises an averaged vibrational spectrum derived from a plurality of normalized and corrected vibrational spectra, wherein the plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region;
(b) deriving fixation features from the obtained test spectral data using a trained fixation estimation engine, wherein the fixation estimation engine is trained using training spectral data sets acquired from a plurality of differentially fixed training biological specimens and wherein the training spectral data sets comprise at least class labels of known fixation durations;
(c) quantitatively determining an estimated fixation duration of the at least partially fixed biological specimen based on the derived fixation features, wherein an amount of time the at least partially fixed test biological specimen has been subjected to a fixation process is predicted.
In some embodiments, the class labels of known fixation durations used during training of the fixation estimation engine are verified through functional IHC testing. In some embodiments, the test biological specimen is unstained. In some embodiments, the best biological specimen is stained for the presence of one or more biomarkers.

In some embodiments, each training spectral data set is derived by: (i) obtaining a training biological specimen; (ii) dividing the obtained training biological specimen into a plurality of training tissue samples; and (iii) fixing each training tissue sample of the plurality of training tissue samples for a different pre-determined amount of time. In some embodiments, the training biological specimens include the same tissue type as the test biological specimen. In some embodiments, the training biological specimens include a different tissue type than the test biological specimen. In some embodiments, the trained fixation status estimation engine includes a machine learning algorithm based on dimensionality reduction. In some embodiments, the dimensionality reduction includes a projection onto latent structure regression model. In some embodiments, the dimensionality reduction includes a principal component analysis. In some embodiments, the trained fixation status estimation engine includes a neural network.

In some embodiments, the obtained test spectral data comprises vibrational spectral information for wavelengths ranging from between about 3200 to about 3400 cm⁻¹. In some embodiments, the obtained test spectral data comprises vibrational spectral information for wavelengths ranging from between about 2800 to about 2900 cm⁻¹. In some embodiments, the obtained test spectral data comprises vibrational spectral information for wavelengths ranging from between about 1020 to about 1100 cm⁻¹. In some embodiments, the obtained test spectral data comprises vibrational spectral information for wavelengths ranging from between about 1520 to about 1580 cm⁻¹.

A third aspect of the present disclosure is a method for predicting a fixation state of an at least partially fixed test biological specimen comprising:
(a) obtaining test spectral data from the at least partially fixed test biological specimen, wherein the obtained test spectral data comprises vibrational spectral data derived from at least a portion of the biological specimen, wherein the obtained test spectral data comprises an averaged vibrational spectrum derived from a plurality of normalized and corrected vibrational spectra, wherein the plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region;
(b) deriving fixation features from the obtained test spectral data using a trained fixation estimation engine, wherein the fixation estimation engine is trained using training spectral data sets acquired from a plurality of differentially fixed training biological specimens;
(c) quantitatively determining an estimated fixation state of the at least partially fixed biological specimen based on the derived fixation features, wherein an amount of time the at least partially fixed test biological specimen has been subjected to a fixation process is predicted.
In some embodiments, the method further includes assessing whether the biological specimen includes a fixation state suitable for labeling with one or more specific binding entities. In some embodiments, the method further includes identifying at least one spectral band within the test data which is positively associated with biological specimen fixation.

In some embodiments, the fixation estimation engine is trained using training spectral data sets acquired from a plurality of differentially fixed training biological specimens. In some embodiments, the training spectral data sets include class labels of known fixation durations, such as known fixation durations determined through functional IHC testing. In some embodiments, the training spectral data sets further include class labels of fixation quality.

In some embodiments, at least two training vibrational spectra are acquired from each individual training biological specimen of the plurality of the training biological specimens, and wherein the at least two sample vibrational spectra are acquired from different portions of the individual training biological specimen. In some embodiments, the at least two different portions of the individual training biological specimen are each treated with one or more fixatives for a different pre-determined amount of time. In some embodiments, the different pre-determined amounts of time range from between about 0 hours to about 24 hours. In some embodiments, the different pre-determined amounts of time range from between about 0 hours to about 12 hours. In some embodiments, at least two training vibrational spectra are each an averaged vibrational spectrum derived from a plurality of normalized and corrected training vibrational spectra.

In some embodiments, the obtained test spectral data includes mid-IR spectral information for at least an amide I band. In some embodiments, the obtained test spectral data comprises vibrational spectral information for wavelengths ranging from between about 3200 to about 3400 cm⁻¹, about 2800 to about 2900 cm⁻¹, about 1020 to about 1100 cm⁻¹, and/or about 1520 to about 1580 cm⁻¹. In some embodiments, the test biological specimen is unstained. In some embodiments, the test biological specimen is stained for the presence of one or more biomarkers.

In some embodiments, the trained fixation status estimation engine includes a machine learning algorithm based on dimensionality reduction. In some embodiments, the dimensionality reduction includes a projection onto latent structure regression model. In some embodiments, the dimensionality reduction includes a principal component analysis. In some embodiments, the trained fixation status estimation engine includes a neural network.

### BRIEF DESCRIPTION OF THE FIGURES

For a general understanding of the features of the disclosure, reference is made to the drawings. In the drawings, like reference numerals have been used throughout to identify identical elements.
FIG. 1 illustrates a representative digital pathology system including an image acquisition device and a computer system in accordance with one embodiment of the present disclosure.
FIG. 2 sets forth various modules that can be utilized in a system or within a digital pathology workflow to estimate a fixation duration of a test tissue sample in accordance with one embodiment of the present disclosure.
FIG. 3 sets forth a flowchart illustrating the various steps of estimate a fixation duration of a test biological specimen using a trained fixation estimation engine in accordance with one embodiment of the present disclosure.
FIGS. 4A - 4C set forth flowcharts illustrating the various steps of acquiring vibrational spectra for a training biological specimen in accordance with one embodiment of the present disclosure.
FIG. 5 sets forth a flowchart illustrating the various steps of acquiring an averaged vibrational spectrum for a test biological specimen in accordance with one embodiment of the present disclosure.
FIG. 6 sets forth a flowchart illustrating the various steps correcting, normalizing, and averaging acquired spectra derived from a biological specimen, including test biological specimens and training biological specimens, in accordance with one embodiments of the present disclosure.
FIG. 7A illustrates typical FR-IR and Raman spectra for collagen.
FIG. 7B provides a table setting forth the infrared and Raman characteristic frequencies of tissue samples.
FIG. 8 provides a graphical illustration of the design of experiment in which 105 individual pieces of tonsil tissue were differentially fixed in neutral buffer formalin for various amounts of time between 0 hour (for example unfixed/ethanol fixed) and 24 hours (fully fixed) in room temperature formalin. Samples were equivalently processed through ethanol and xylene and embedded in paraffin. One slide from each tissue block was stained for BCL2, ki-67, and FOXP3 and two cuts from each block were imaged spectroscopically with the mid-IR microscope.
FIGS. 9A and 9B provide overview of brightfield IHC imaging and image processing for biomarker quantitation.

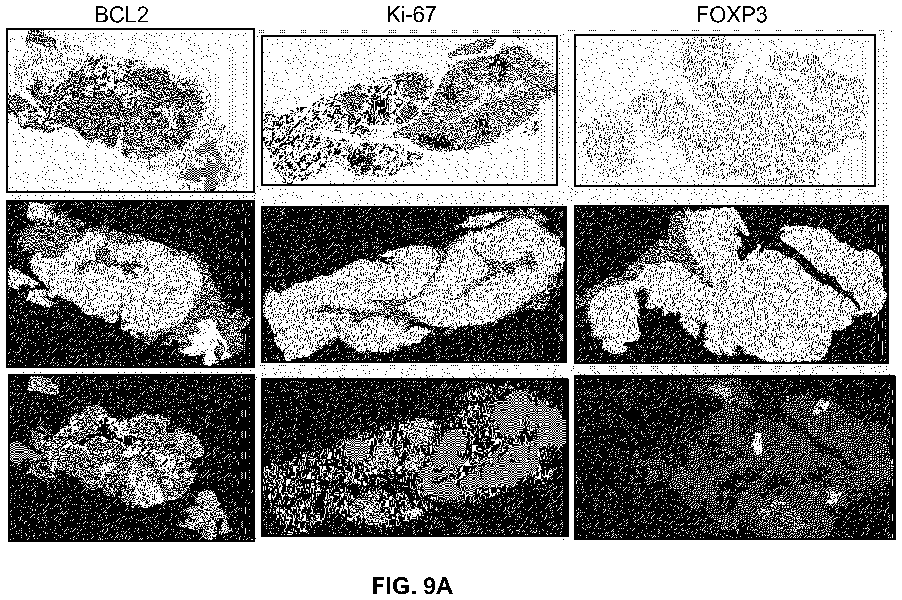

FIG. 9A illustrates imaging and image segmentations algorithm on whole slide scan. Top row: Original brightfield renditions of whole slide scan for each of the three antigens. Middle Row: Results of image segmentation algorithm, dark grey areas were excluded from digital analysis (stroma, connective tissue, etc.) and light grey regions were included in analysis. Bottom Row: Hot spot rendering of biomarker expression level. Dark grey = high expression density, very dark grey = low expression density, black = negative/no tissue.

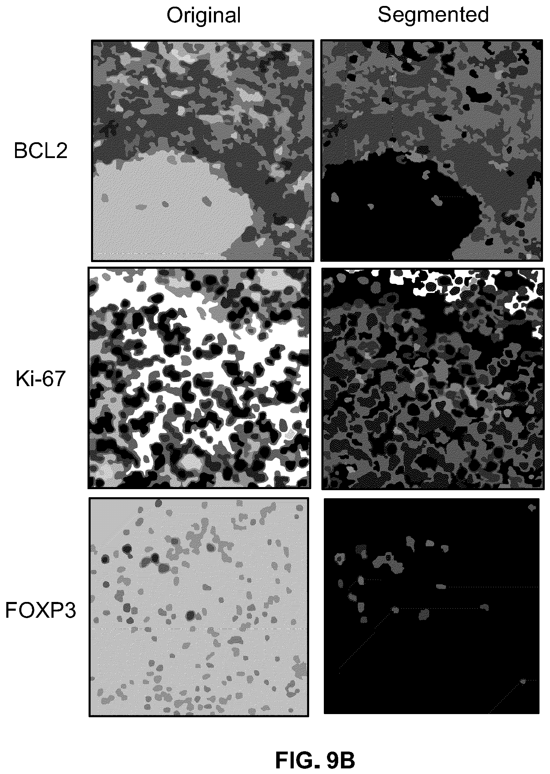

FIG. 9B provides an example of staining for all three antigens at 20X including the original image (left column) and the segmented image (right column).

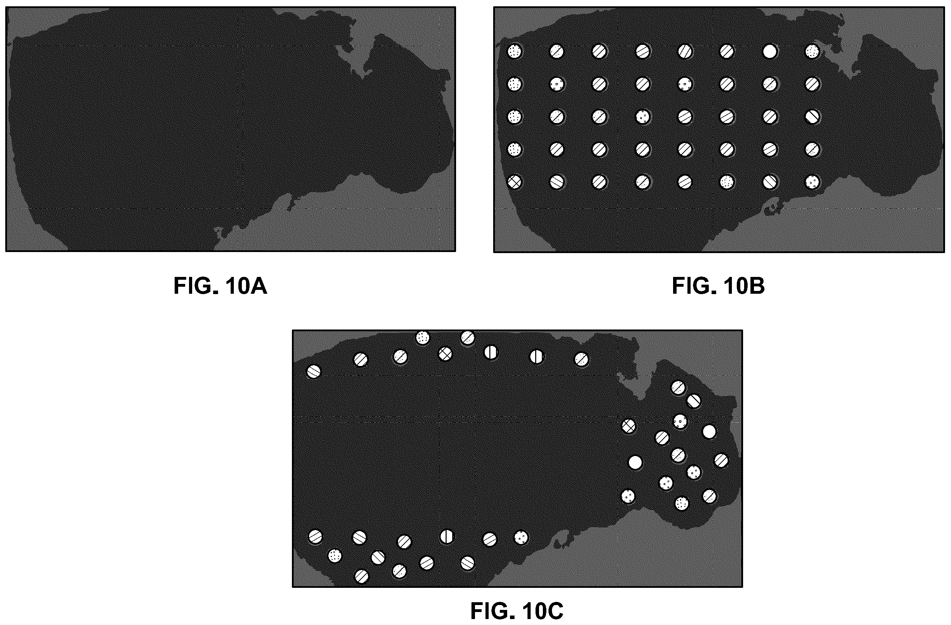

FIGS. 10A - 10H provide an overview of mid-IR collection.
FIG. 10A provides a brightfield image of tissue sample acquired on Bruker Hyperion 3000 mid-IR microscope.
FIGS. 10B and 10C illustrate regions of the sample that were spectroscopically imaged are indicated with the colored circles.

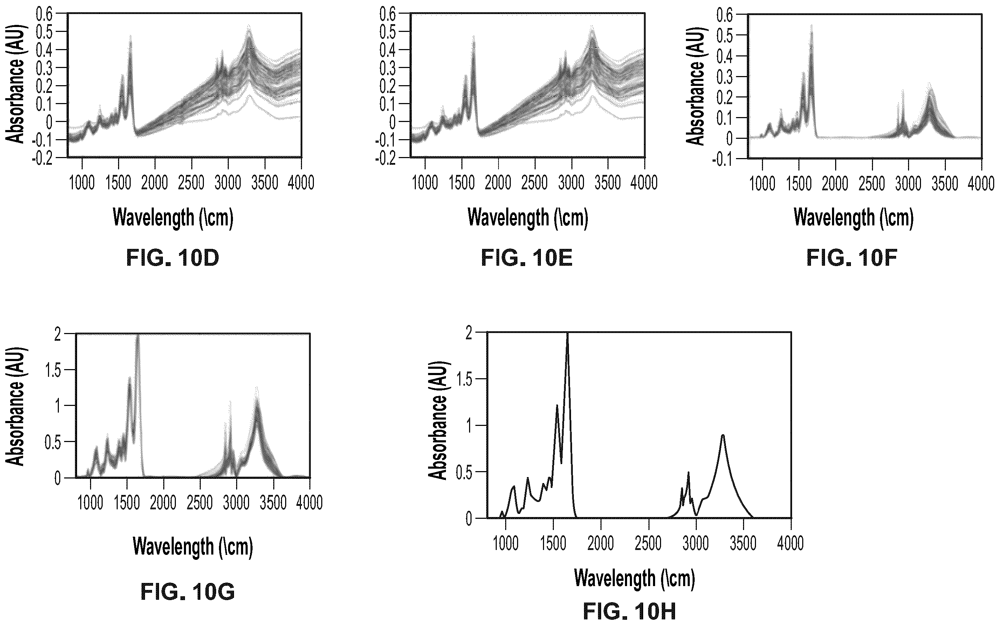

FIG. 10D provides an original mid-IR spectrum of all points imaged within the tissue. Each line represents the spectrum of one circle in FIG. 10B and FIG. 10 C.
FIG. 10E provides mid-IR spectra after atmospheric correction.
FIG. 10F provides mid-IR spectra after baseline correction to mitigate the effects of scattering.
FIG. 10G provides mid-IR spectra after amplitude normalization.
FIG. 10H provides spatially averaged mid-IR spectra, representing the average mid-IR spectra of the entire tissue sample.

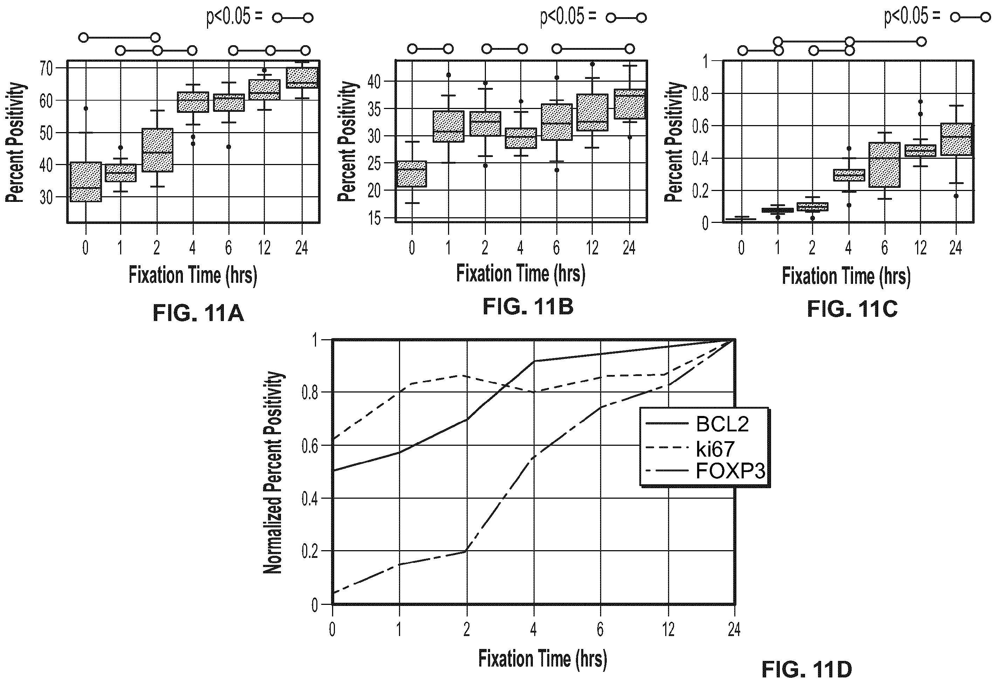

FIGS. 11A - C set forth a quantitative analysis of IHC expression of BCL2 (FIG. 11A), ki-67 (FIG. 11B), and FOXP3 (FIG. 11C).
FIG. 11D illustrates a plot of IHC expression for all three biomarkers versus fixation time in which the mean expression is plotted on a normalized scale so relative changes in each biomarker versus fixation time can be observed Bars represent significant levels of p<0.05 as determined by a double-sided ranksum test.

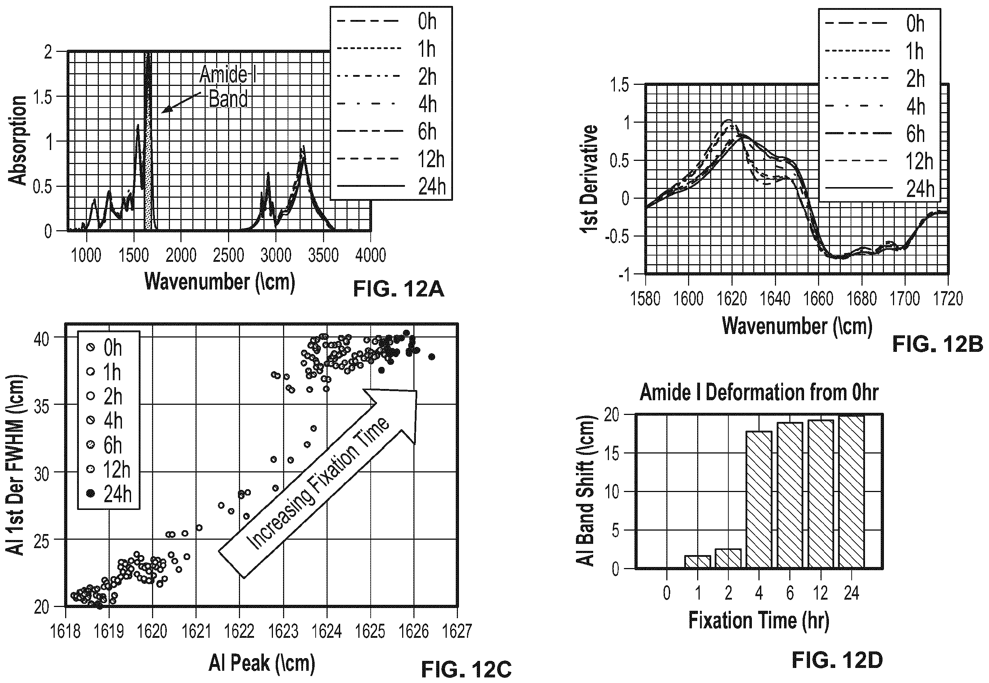

FIG. 12A provides an average mid-IR absorption for all fixation times, with the approximate location of the Amide I band indicated.
FIG. 12B provides a mid-IR absorption of the Amide I band. Solid lines represent average absorption and error bars represents plus/minus standard deviation of all tissues.
FIG. 12C provides quantitative view of deformation of Amide I band in which the peak location of the band is plotted versus the full width at half maximum ("FWHM").
FIG. 12D provides an average shift of Amide 1 band relative to the peak location and FWHM of fixation time = 0.

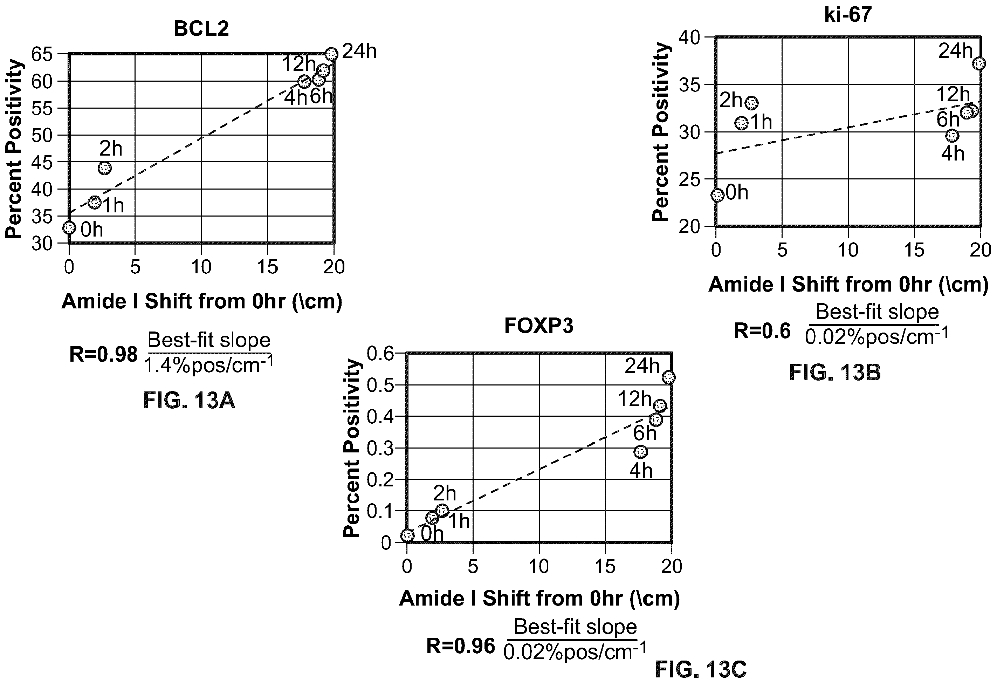

FIGS. 13A - 13C illustrate plots of the absolute Amide I shift versus IHC expression for BCL2 (FIG. 13A), ki-67 (FIG. 13B), and FOXP3 (FIG. 13C).

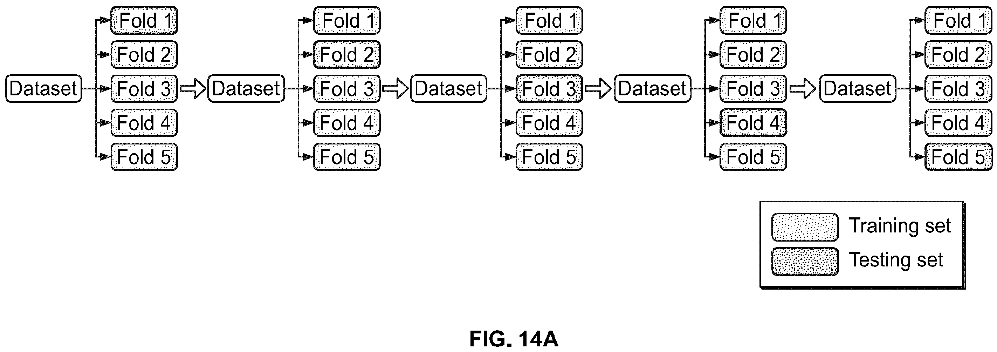

FIG. 14A illustrates K-fold cross validation.

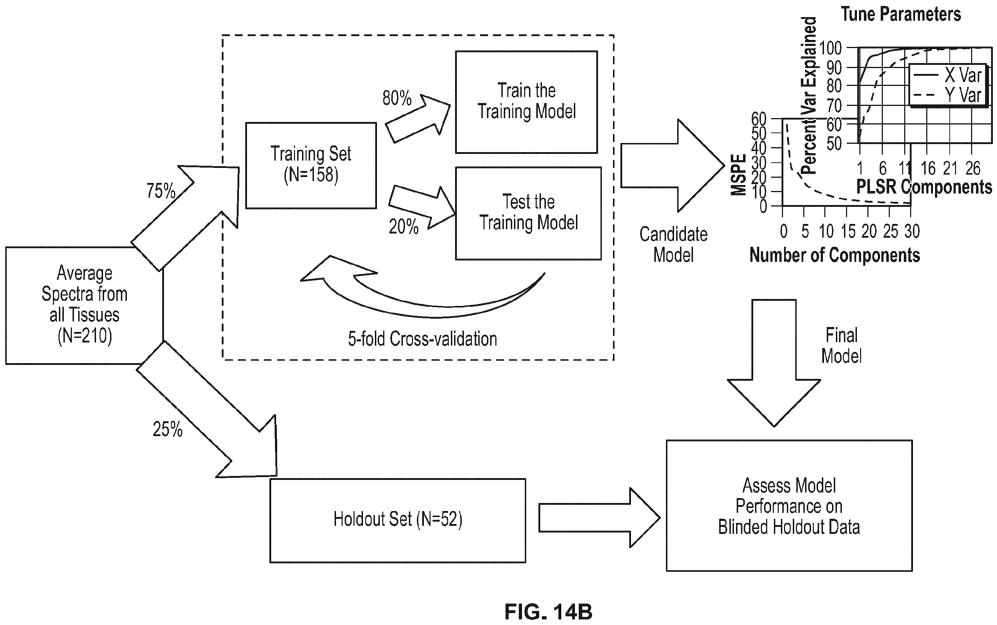

FIG. 14B sets forth a flow chart of training and validating of the projection onto latent structure regression algorithm.

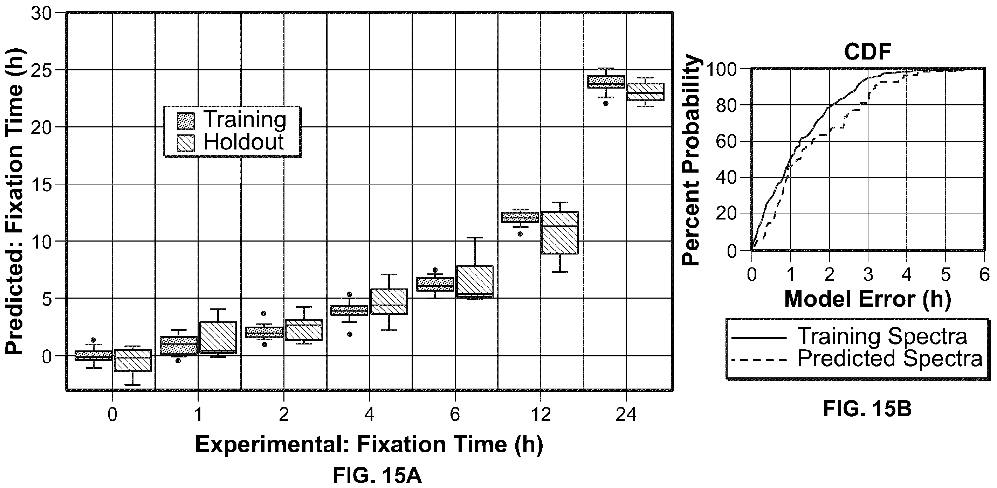

FIG. 15A provides the results of fixation prediction model on training data (left) and holdout blinded tissue samples (right).
FIG. 15B provides the cumulative distribution function (CDF) for the spectra from the training set and the blinded spectra. The developed model was able to predict the fixation time of unknown samples to 1.4 hours on average.

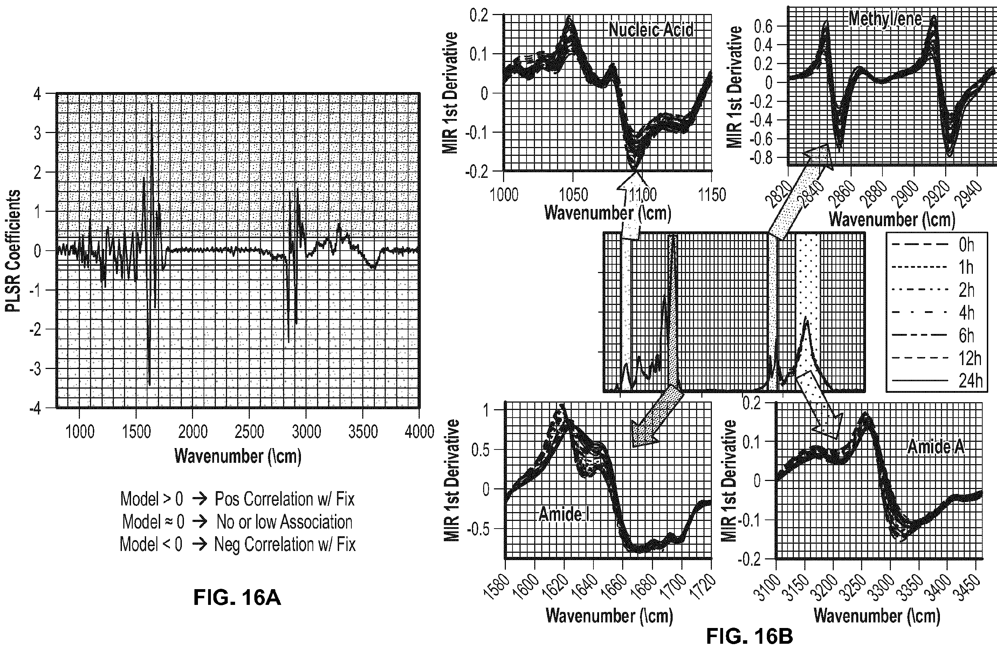

FIG. 16A sets forth the weights from a developed PLSR model with positive coefficients representing a positive predictor of fixation time and negative coefficients representing a negative predictor of fixation time.
FIG. 16B sets forth the mid-IR spectra of four bands that showed differences based on how long a sample has been fixed in formalin.

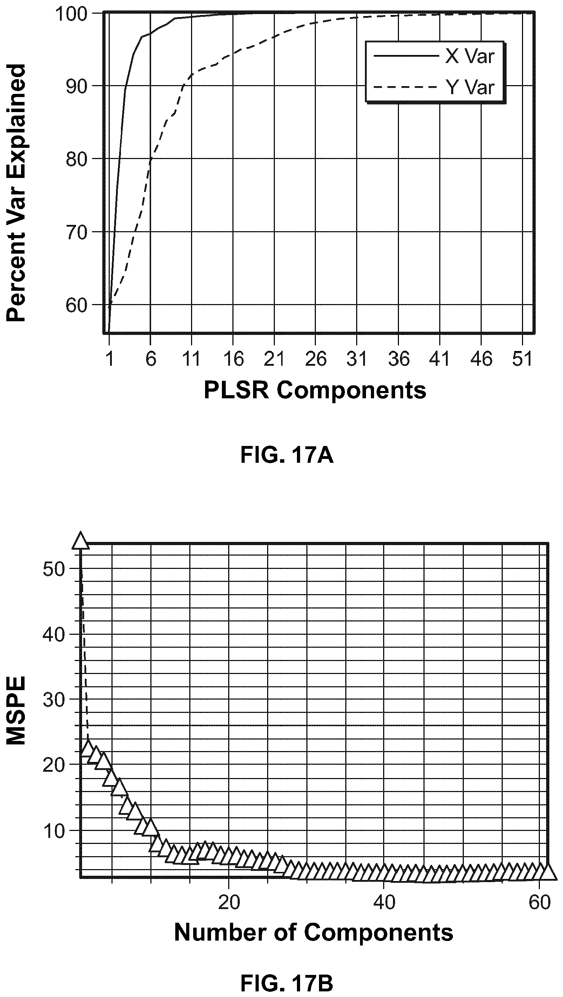

FIG. 17A provides a graph of the percent of variance in the X and Y variables that are explained by increasing numbers of components in the predictive model.
FIG. 17B provides a graph of the mean squared predictive error (MSPE) of the model from the cross-validation holdout datasets with increasing number of components. The first ~20 components significantly increase the model's predictive accuracy and adding components beyond 20 results in little improvement predictive power.

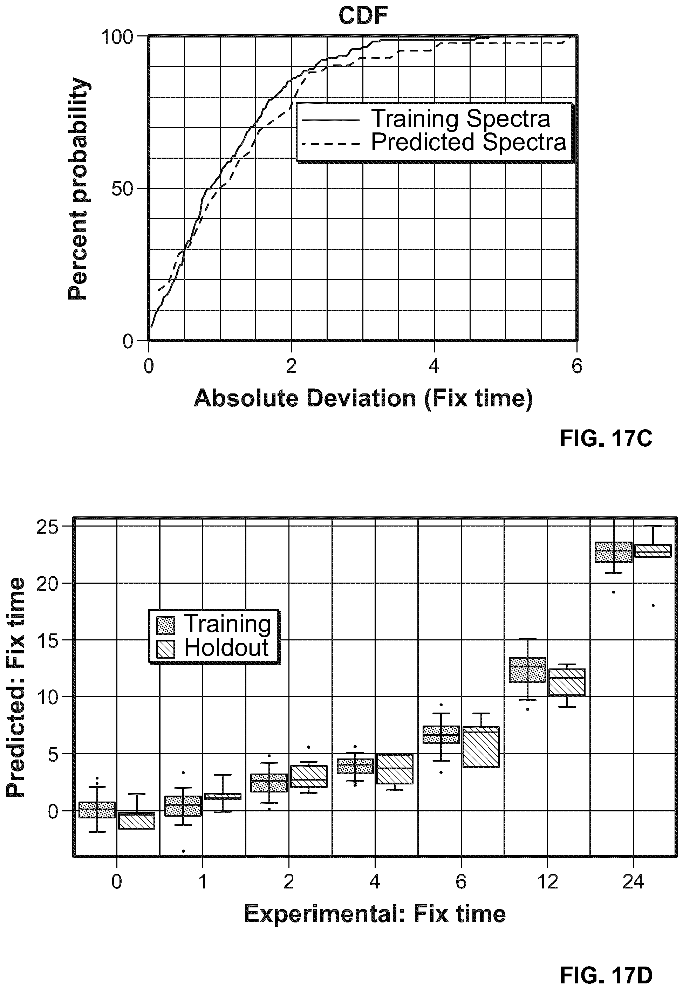

FIG. 17C provides a graph which illustrates the performance of the model on the training set and the hold-out validation data. The predictive error is identical for both types of data indicating a well-trained model that has identified the true signature of retrieval in the mid-IR spectra.
FIG. 17D provides a box and whisker graph of a model's performance by displaying the experimental fixation time versus the model predicted time for both the training tissues (left boxes) and validation tissues (right boxes).

### DETAILED DESCRIPTION

It should also be understood that, unless clearly indicated to the contrary, in any methods claimed herein that include more than one step or act, the order of the steps or acts of the method is not necessarily limited to the order in which the steps or acts of the method are recited.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described.

As used herein, the singular terms "a," "an," and "the" include plural referents unless context clearly indicates otherwise. Similarly, the word "or" is intended to include "and" unless the context clearly indicates otherwise. The term "includes" is defined inclusively, such that "includes A or B" means including A, B, or A and B.

As used herein in the specification and in the claims, "or" should be understood to have the same meaning as "and/or" as defined above. For example, when separating items in a list, "or" or "and/or" shall be interpreted as being inclusive, for example, the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (for example "one or the other but not both") when preceded by terms of exclusivity, such as "either," "one of," "only one of" or "exactly one of." "Consisting essentially of," when used in the claims, shall have its ordinary meaning as used in the field of patent law.

The terms "comprising," "including," "having," and the like are used interchangeably and have the same meaning. Similarly, "comprises," "includes," "has," and the like are used interchangeably and have the same meaning. Specifically, each of the terms is defined consistent with the common United States patent law definition of "comprising" and is therefore interpreted to be an open term meaning "at least the following," and is also interpreted not to exclude additional features, limitations, aspects, etc. Thus, for example, "a device having components a, b, and c" means that the device includes at least components a, b, and c. Similarly, the phrase: "a method involving steps a, b, and c" means that the method includes at least steps a, b, and c. Moreover, while the steps and processes may be outlined herein in a particular order, the skilled artisan will recognize that the ordering steps and processes may vary.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

As used herein, the term "biological specimen," "sample," or "tissue sample" refers to any sample including a biomolecule (such as a protein, a peptide, a nucleic acid, a lipid, a carbohydrate, or a combination thereof) that is obtained from any organism including viruses. Other examples of organisms include mammals (such as humans; veterinary animals like cats, dogs, horses, cattle, and swine; and laboratory animals like mice, rats, and primates), insects, annelids, arachnids, marsupials, reptiles, amphibians, bacteria, and fungi. Biological specimens include tissue samples (such as tissue sections and needle biopsies of tissue), cell samples (such as cytological smears such as Pap smears or blood smears or samples of cells obtained by microdissection), or cell fractions, fragments or organelles (such as obtained by lysing cells and separating their components by centrifugation or otherwise). Other examples of biological specimens include blood, serum, urine, semen, fecal matter, cerebrospinal fluid, interstitial fluid, mucous, tears, sweat, pus, biopsied tissue (for example, obtained by a surgical biopsy or a needle biopsy), nipple aspirates, cerumen, milk, vaginal fluid, saliva, swabs (such as buccal swabs), or any material containing biomolecules that is derived from a first biological specimen. In certain embodiments, the term "biological specimen" as used herein refers to a sample (such as a homogenized or liquefied sample) prepared from a tumor or a portion thereof obtained from a subject.

As used herein, the terms "biomarker" or "marker" refer to a measurable indicator of some biological state or condition. In particular, a biomarker may be a protein or peptide, e.g. a surface protein, that can be specifically stained, and which is indicative of a biological feature of the cell, e.g. the cell type or the physiological state of the cell. An immune cell marker is a biomarker that is selectively indicative of a feature that relates to an immune response of a mammal. A biomarker may be used to determine how well the body responds to a treatment for a disease or condition or if the subject is predisposed to a disease or condition. In the context of cancer, a biomarker refers to a biological substance that is indicative of the presence of cancer in the body. A biomarker may be a molecule secreted by a tumor or a specific response of the body to the presence of cancer. Genetic, epigenetic, proteomic, glycomic, and imaging biomarkers can be used for cancer diagnosis, prognosis, and epidemiology. Such biomarkers can be assayed in non-invasively collected biofluids like blood or serum. Several gene and protein based biomarkers have already been used in patient care including but, not limited to, AFP (Liver Cancer), BCR- ABL (Chronic Myeloid Leukemia), BRCA1 / BRCA2 (Breast/Ovarian Cancer), BRAF V600E (Melanoma/Colorectal Cancer), CA-125 (Ovarian Cancer), CA19.9 (Pancreatic Cancer), CEA (Colorectal Cancer), EGFR (Non-small-cell lung carcinoma), HER-2 (Breast Cancer), KIT (Gastrointestinal stromal tumor), PSA (Prostate Specific Antigen), S100 (Melanoma), and many others. Biomarkers may be useful as diagnostics (to identify early stage cancers) and/or prognostics (to forecast how aggressive a cancer is and/or predict how a subject will respond to a particular treatment and/or how likely a cancer is to recur).

As used herein, the term "cell," refers to a prokaryotic cell or a eukaryotic cell. The cell may be an adherent or a non-adherent cell, such as an adherent prokaryotic cell, adherent eukaryotic cell, non-adherent prokaryotic cell, or non-adherent eukaryotic cell. A cell may be a yeast cell, a bacterial cell, an algae cell, a fungal cell, or any combination thereof. A cell may be a mammalian cell. A cell may be a primary cell obtained from a subject. A cell may be a cell line or an immortalized cell. A cell may be obtained from a mammal, such as a human or a rodent. A cell may be a cancer or tumor cell. A cell may be an epithelial cell. A cell may be a red blood cell or a white blood cell. A cell may be an immune cell such as a T cell, a B cell, a natural killer (NK) cell, a macrophage, a dendritic cell, or others. A cell may be a neuronal cell, a glial cell, an astrocyte, a neuronal support cell, a Schwann cell, or others. A cell may be an endothelial cell. A cell may be a fibroblast or a keratinocyte. A cell may be a pericyte, hepatocyte, a stem cell, a progenitor cell, or others. A cell may be a circulating cancer or tumor cell or a metastatic cell. A cell may be a marker specific cell such as a CD8+ T cell or a CD4+ T cell. A cell may be a neuron. A neuron may be a central neuron, a peripheral neuron, a sensory neuron, an interneuron, a intraneuronal, a motor neuron, a multipolar neuron, a bipolar neuron, or a pseudo-unipolar neuron. A cell may be a neuron supporting cell, such as a Schwann cell. A cell may be one of the cells of a blood-brain barrier system. A cell may be a cell line, such as a neuronal cell line. A cell may be a primary cell, such as cells obtained from a brain of a subject. A cell may be a population of cells that may be isolated from a subject, such as a tissue biopsy, a cytology specimen, a blood sample, a fine needle aspirate (FNA) sample, or any combination thereof. A cell may be obtained from a bodily fluid such as urine, milk, sweat, lymph, blood, sputum, amniotic fluid, aqueous humor, vitreous humor, bile, cerebrospinal fluid, chyle, chyme, exudates, endolymph, perilymph, gastric acid, mucus, pericardial fluid, peritoneal fluid, pleural fluid, pus, rheum, saliva, sebum, serous fluid, smegma, sputum, tears, vomit, or other bodily fluid. A cell may comprise cancerous cells, non-cancerous cells, tumor cells, non-tumor cells, healthy cells, or any combination thereof.

As used herein, the term "cytological sample" refers to a cellular sample in which the cells of the sample have been partially or completely disaggregated, such that the sample no longer reflects the spatial relationship of the cells as they existed in the subject from which the cellular sample was obtained. Examples of cytological samples include tissue scrapings (such as a cervical scraping), fine needle aspirates, samples obtained by lavage of a subject, et cetera.

As used herein, the term "fixation" refers to a process by which molecular and/or morphological details of a cellular sample are preserved. There are generally three kinds of fixation processes: (1) heat fixation, (2) perfusion; and (3) immersion. With heat fixation, samples are exposed to a heat source for a sufficient period of time to heat kill and adhere the sample to the slide. Perfusion involves use of the vascular system to distribute a chemical fixative throughout a whole organ or a whole organism. Immersion involves immersing a sample in a volume of a chemical fixative and allowing the fixative to diffuse throughout the sample. Chemical fixation involves diffusion or perfusion of a chemical throughout the cellular samples, where the fixative reagent causes a reaction that preserves structures (both chemically and structurally) as close to that of living cellular sample as possible. Chemical fixatives can be classified into two broad classes based on mode of action: cross-linking fixatives and non-cross-linking fixatives. Cross-linking fixatives - typically aldehydes - create covalent chemical bonds between endogenous biological molecules, such as proteins and nucleic acids, present in the tissue sample. Formaldehyde is the most commonly used cross-linking fixative in histology. Formaldehyde may be used in various concentrations for fixation, but it primarily is used as 10% neutral buffered formalin (NBF), which is about 3.7% formaldehyde in an aqueous phosphate buffered saline solution. Paraformaldehyde is a polymerized form of formaldehyde, which depolymerizes to provide formalin when heated. Glutaraldehyde operates in similar manner as formaldehyde, but is a larger molecule having a slower rate of diffusion across membranes. Glutaraldehyde fixation provides a more rigid or tightly linked fixed product, causes rapid and irreversible changes, fixes quickly and well at 4 °C, provides good overall cytoplasmic and nuclear detail, but is not ideal for immunohistochemistry staining. Some fixation protocols use a combination of formaldehyde and glutaraldehyde. Glyoxal and acrolein are less commonly used aldehydes. Denaturation fixatives - typically alcohols or acetone - act by displacing water in the cellular sample, which destabilizes hydrophobic and hydrogen bonding within proteins. This causes otherwise water-soluble proteins to become water insoluble and precipitate, which is largely irreversible.

As used herein, the term "immunohistochemistry" refers to a method of determining the presence or distribution of an antigen in a sample by detecting interaction of the antigen with a specific binding agent, such as an antibody. A sample is contacted with an antibody under conditions permitting antibody-antigen binding. Antibody-antigen binding can be detected by means of a detectable label conjugated to the antibody (direct detection) or by means of a detectable label conjugated to a secondary antibody, which binds specifically to the primary antibody (indirect detection). In some instances, indirect detection can include tertiary or higher antibodies that serve to further enhance the detectability of the antigen. Examples of detectable labels include enzymes, fluorophores and haptens, which in the case of enzymes, can be employed along with chromogenic or fluorogenic substrates.

As used herein, the terms "multi-channel image" or "multiplex image" encompasses a digital image obtained from a biological tissue sample in which different biological structures, such as nuclei, cells, and tissue structures, are simultaneously stained with specific fluorescent dyes, quantum dots, chromogens, etc., each of which fluoresces or are otherwise detectable in a different spectral band thus constituting one of the channels of the multi-channel image.

As used herein, the term "slide" refers to any substrate (e.g., substrates made, in whole or in part, glass, quartz, plastic, silicon, etc.) of any suitable dimensions on which a biological specimen is placed for analysis, and more particularly to a "microscope slide" such as a standard 3 inch by 1 inch microscope slide or a standard 75 mm by 25 mm microscope slide. Examples of biological specimens that can be placed on a slide include, without limitation, a cytological smear, a thin tissue section (such as from a biopsy), and an array of biological specimens, for example a tissue array, a cellular array, a DNA array, an RNA array, a protein array, or any combination thereof. Thus, in one embodiment, tissue sections, DNA samples, RNA samples, and/or proteins are placed on a slide at particular locations. In some embodiments, the term slide may refer to SELDI and MALDI chips, and silicon wafers.

As used herein the term "specific binding entity" refers to a member of a specific-binding pair. Specific binding pairs are pairs of molecules that are characterized in that they bind each other to the substantial exclusion of binding to other molecules (for example, specific binding pairs can have a binding constant that is at least 10³ M⁻¹ greater, 10⁴ M⁻¹ greater or 10⁵ M⁻¹ greater than a binding constant for either of the two members of the binding pair with other molecules in a tissue sample). Particular examples of specific binding moieties include specific binding proteins (for example, antibodies, lectins, avidins such as streptavidins, and protein A). Specific binding moieties can also include the molecules (or portions thereof) that are specifically bound by such specific binding proteins.

As used herein, the term "spectra data" encompasses raw image spectral data acquired from a biological specimen or any portion thereof, such as with a spectrometer.

As used herein, the term "spectrum" refers to information (absorption, transmission, reflection) obtained "at" or within a certain wavelength or wavenumber range of electromagnetic radiation. A wavenumber range can be as large as 4000 cm-1 or as narrow as 0.01 cm-1. Note that a measurement at a so-called "single laser wavelength" will typically cover a small spectral range (e.g., the laser linewidth) and will hence be included whenever the term "spectrum" is used throughout this manuscript. A transmission measurement at a fixed wavelength setting of a quantum cascade laser, for example, shall hereby fall under the term spectrum throughout this application.

As used herein, the term "substantially" means the qualitative condition of exhibiting total or near-total extent or degree of a characteristic or property of interest. In some embodiments, "substantially" means within about 5%. In some embodiments, "substantially" means within about 10%. In some embodiments, "substantially" means within about 15%. In some embodiments, "substantially" means within about 20%.

As used herein, the term "tissue sample" or "tissue specimen" (used interchangeably herein) shall refer to a cellular sample that preserves the cross-sectional spatial relationship between the cells as they existed within the subject from which the sample was obtained. "Tissue sample" shall encompass both primary tissue samples (for example cells and tissues produced by the subject) and xenografts (for example foreign cellular samples implanted into a subject).

### OVERVIEW

It is believed that fixation quality, and hence fixation duration, may have an impact on downstream analytical methods. For example, under current clinical practice, it is important to control the tissue fixation duration to achieve a compromise between the preservation of tissue morphology and the loss of antigenicity. Indeed, a fixation duration that is too short or too long may negatively impact downstream sample processing. Thus, there remains a need for the accurate prediction of fixation duration of a sample prior to downstream processing, e.g. prior to contacting the sample with one or more unmasking agents or prior to contacting the same with one or more specific binding entities.

The present disclosure describes systems and methods for quantitatively estimating a fixation duration of a tissue sample treated with one or more fixatives. For example, the present disclosure provides systems and methods for predicting the amount of time a biological specimen has been subjected to a fixation process, e.g. about 0 hours, about 1 hour, about 2 hours, about 4 hours, about 12 hours, about 16 hours, about 24 hours, about 48 hours, etc. The present disclosure also describes systems and methods for training a fixation estimation engine to enable a quantitative determination of a fixation duration based on ground truth data. In some embodiments, the present disclosure also provides for systems and methods for providing a qualitative estimate of the fixation quality of a biological specimen.

At least some embodiments of the present disclosure relate to computer systems and methods for analyzing spectral data acquired from biological specimens which have at least been subjected to a fixation process for either a known period of time, an estimated period of time, or an unknown period of time. For example, and in the case of spectra data acquired to train a fixation estimation engine, the fixation duration of a training biological specimen may be known (and/or verified through functional IHC testing, as described herein). By way of another example, and in the case of test spectra derived from a subject's biological specimen (for example a test biological specimen), the fixation duration may be unknown or roughly estimated. In accordance with the present disclosure, a trained fixation estimation engine may be used to provide a quantitative estimate of the fixation duration of the test biological specimen where the fixation duration is unknown or only roughly estimated. Additionally, the trained fixation estimation engine may be used to verify a fixation duration of a test biological specimen that was subjected to one or more fixation processes for unknown amounts of time. For example, if a received test biological specimen includes a notation that the specimen was fixed for 10 hours, the systems and methods of the present disclosure may be used to verify the fixation duration noted. In this manner, an assessment of a sample may be made so as to determine whether the sample is ready for downstream processing and/or analysis, e.g. whether the test biological specimen is in a fixed state suitable for labeling with a particular specific binding entity.

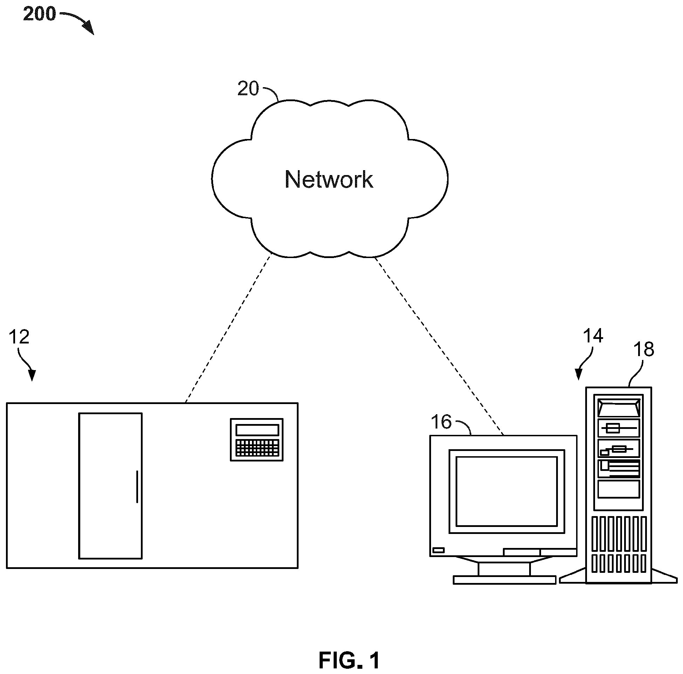

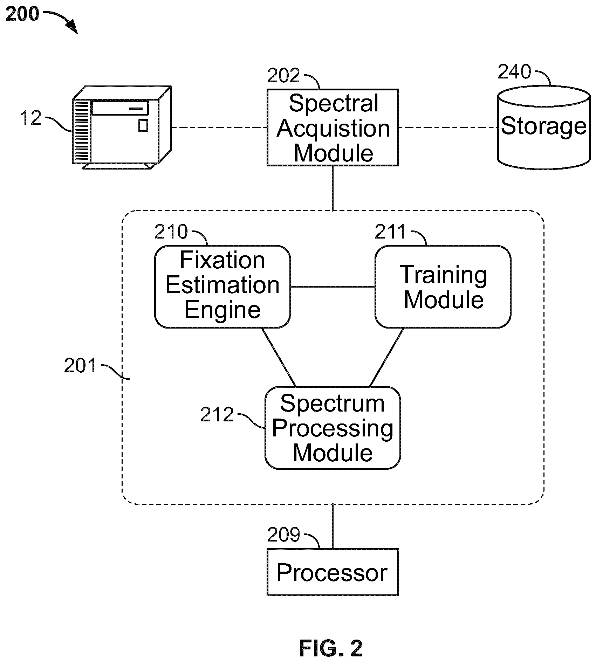

A system 200 for acquiring vibrational spectra data, e.g. mid-infrared (mid-IR) spectral data or Raman spectral data, and analyzing biological specimens (including test biological specimens and training biological specimens) is illustrated in FIGS. 1 and 2. The system may include a spectral acquisition device 12, such as one configured to acquire a vibrational spectrum of a biological specimen (or any portion thereof), and a computer 14, whereby the spectral acquisition device 12 and computer may be communicatively coupled together (e.g. directly, or indirectly over a network 20). The computer system 14 can include a desktop computer, a laptop computer, a tablet, or the like, digital electronic circuitry, firmware, hardware, memory 201, a computer storage medium (240), a computer program or set of instructions (e.g. where the program is stored within the memory or storage medium), one or more processors (209) (including a programmed processor), and any other hardware, software, or firmware modules or combinations thereof (such as described further herein). For example, the system 14 illustrated in FIG. 1 may include a computer with a display device 16 and an enclosure 18. The computer system can store acquired spectral data locally, such as in a memory, on a server, or another network connected device.

Vibrational spectroscopy is concerned with the transitions due to absorption or emission of electromagnetic radiation. These transitions are believed to appear in the range of 102 to 104 cm⁻¹ and originate from the vibration of nuclei constituting the molecules in any given sample. It is believed that a chemical bond in a molecule can vibrate in many ways, and each vibration is called vibrational mode. There are two types of molecular vibrations, stretching and bending. A stretching vibration is characterized by movement along the bond axis with increasing or decreasing of the interatomic distances, whereas a bending vibration consists of a change in bond angles with respect to the remainder of the molecule. The two widely used spectroscopic techniques based on vibrational energy are the Raman spectroscopy and the infrared spectroscopy. Both methods give complementary information and are based on the fact that within any molecules the atoms vibrate with a few definite sharply defined frequency characteristics of that molecule. When a sample is irradiated with a beam of incident radiation, it absorbs energy at frequencies characteristic to that of the frequency of the vibration of chemical bonds present in the molecules. This absorption of energy through the vibration of chemical bond results in an infrared spectrum.

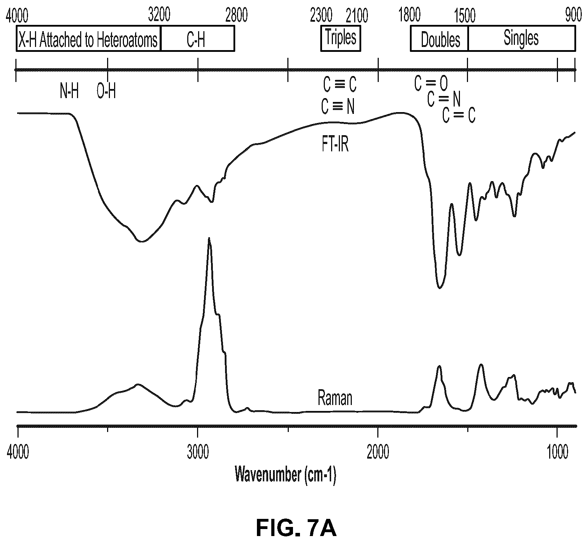

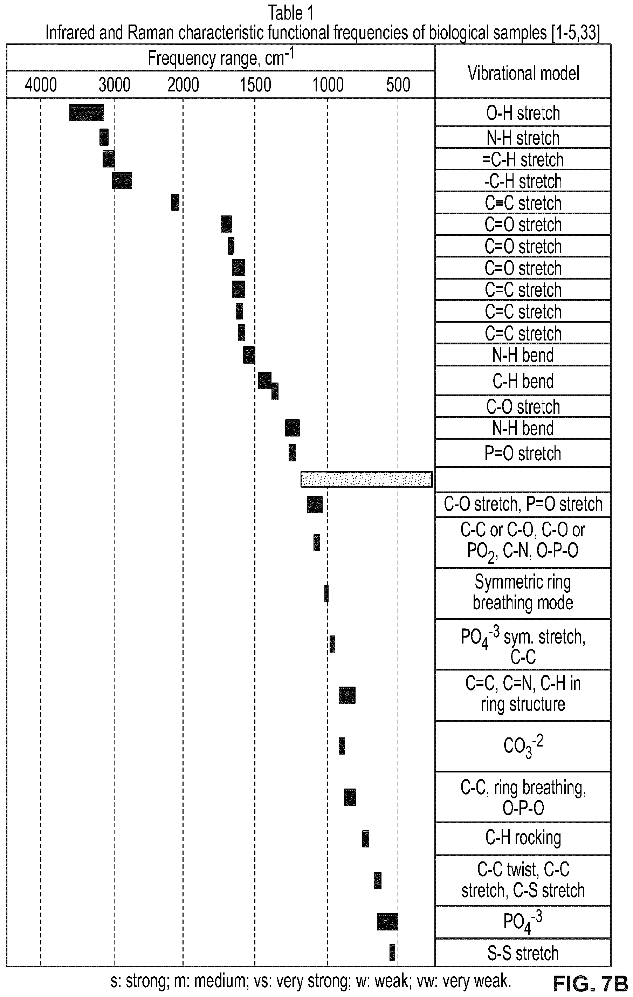

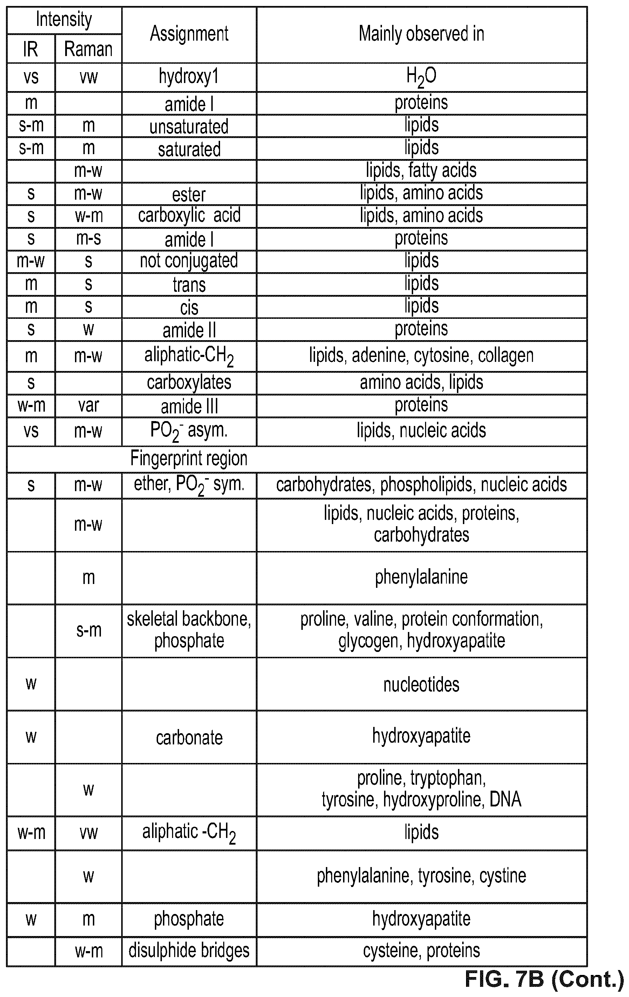

Although IR and Raman spectroscopies measure the vibrational energies of molecules, both methods are dependent on different selection rules, for example, an absorption process and a scattering effect. Although their contrast mechanisms are different and each methodology has respective strengths and weaknesses, the resultant spectra from each modality are often correlated (see, e.g. FIGS. 7A and 7B).

IR spectroscopy is based on the absorption of electromagnetic radiation, whereas Raman spectroscopy relies upon inelastic scattering of electromagnetic radiation. Infrared spectroscopy offers a number of analytical tools, from absorption to reflection and dispersion techniques, extended in a large range of wave numbers and including the near, middle, and far infrared regions in which the different bonds present in the sample molecules offer numerous generic and characteristic bands suitable to be employed for both qualitative and quantitative purposes. The sample is radiated by IR light in IR spectroscopy, and the vibrations induced by electrical dipole moment are detected.

Raman spectroscopy is a scattering phenomenon and arises due to the difference between the incident and scattered radiation frequencies. It utilizes scattered light to gain knowledge about molecular vibration, which can provide information regarding the structure, symmetry, electronic environment, and bonding of the molecule. In Raman spectroscopy, the sample is illuminated by a monochromatic visible or near IR light from a laser source and its vibrations during the electrical polarizability changes are determined.

Any spectral acquisition device may be utilized in the systems of the present disclosure. Examples of suitable spectral acquisition devices or components of such devices for use in acquiring mid-infrared spectra are described in United States Patent Publication Nos.: 2018/0109078a and 2016/0091704; and in US Patent Nos.: 10,041,832, 8,036,252, 9,046,650, 6,972,409, and 7,280,576.

Any method suitable for generating a representative mid-IR spectrum for the samples can be used. Fourier-transform Infrared Spectroscopy and its biomedical applications are discussed in, for example, in P. Lasch, J. Kneipp (Eds.) Biomedical Vibrational Spectroscopy" 2008 (John Wiley&Sons). More recently, however, tunable quantum cascade lasers have enabled the rapid spectroscopy and microscopy of biomedical specimen (see N. Kröger et al., in: Biomedical Vibrational Spectroscopy VI: Advances in Research and Industry, edited by A. Mahadevan-Jansen, W. Petrich, Proc. of SPIE Vol. 8939, 89390Z; N. Kröger et al., J. Biomed. Opt. 19 (2014) 111607; N. Kröger-Lui et al., Analyst 140 (2015) 2086) by virtue of their high spectral power density. It is believed that this work constitutes a major breakthrough (as compared to foregoing Infrared microscopy setups) towards applicability in that the investigation is much faster (e.g. 5 minutes instead of 18 hours), does not need liquid nitrogen cooling and provides more many more pixels per image at substantially lower cost. One particular advantage of QCL-based microscopy in the context of the quality assessment of unstained tissue is the larger field of view (as compared to FT-IR imaging) which is enabled by the microbolometer array detector with e.g. 640 x 480 pixels.

In some embodiments, spectra may be obtained over broad wavelength ranges, one or more narrow wavelength ranges, or even at merely a single wavelength, or a combination thereof. For example, spectra may be acquired for an Amide I band and Amide II band. By way of another example, the spectra may be acquired over a wavelength ranging from about 3200 to about 3400 cm⁻¹, about 2800 to about 2900 cm⁻¹, about 1020 to about 1100 cm⁻¹, and/or about 1520 to about 1580 cm⁻¹. In some embodiments, the spectra may be acquired over a wavelength ranging from about 3200 to about 3400 cm⁻¹. In some embodiments, the spectra may be acquired over a wavelength ranging from about 2800 to about 2900 cm⁻¹.In some embodiments, the spectra may be acquired over a wavelength ranging from about 1020 to about 1100 cm⁻¹. In some embodiments, the spectra may be acquired over a wavelength ranging from about 1520 to about 1580 cm⁻¹. Narrowing down the spectral range is usually advantageous in terms of the acquisition speed, especially when using quantum cascade lasers. In one particular embodiment, a single tunable laser is tuned to the respective wavelengths one after the other. Alternatively, a set of non-tunable lasers at fixed frequency could be used such that the wavelength selection is done by switching on and off whichever laser is needed for a measurement at a particular frequency.

The spectra may be acquired using, for example, transmission or reflection measurements. For transmission measurements, barium fluorite, calcium fluoride, silicon, thin polymer films, or zinc selenide are usually used as substrate. For the reflection measurements, gold- or silver-plated substrates are common as well as standard microscope glass slides, or glass slides which are coated with a mid-IR-reflection coating (e.g. multilayer dielectric coating or thin sliver-coating). In addition, means for using surface enhancement (e.g. SEIRS) may be implemented such as structured surfaces like nanoantennas.

The skilled artisan will appreciate that other computer devices or systems may be utilized and that the computer systems described herein may be communicatively coupled to additional components, e.g. microscopes, imaging devices, scanner, other imaging systems, automated slide preparation equipment, etc. Some of these additional components and the various computers, networks, etc. that may be utilized are described further herein.

For example, in some embodiments the system 200 may further include an imaging device and images captured from the imaging device may be stored in binary form, such as locally or on a server. The digital images can also be divided into a matrix of pixels. The pixels can include a digital value of one or more bits, defined by the bit depth. In general, the imaging apparatus (or other image source including pre-scanned images stored in a memory) can include, without limitation, one or more image capture devices. Image capture devices can include, without limitation, a camera (e.g., an analog camera, a digital camera, etc.), optics (e.g., one or more lenses, sensor focus lens groups, microscope objectives, etc.), imaging sensors (e.g., a charge-coupled device (CCD), a complimentary metal-oxide semiconductor (CMOS) image sensor, or the like), photographic film, or the like. In digital embodiments, the image capture device can include a plurality of lenses that cooperate to prove on-the-fly focusing. An image sensor, for example, a CCD sensor can capture a digital image of the specimen. In some embodiments, the imaging apparatus is a brightfield imaging system, a multispectral imaging (MSI) system or a fluorescent microscopy system. The digitized tissue data may be generated, for example, by an image scanning system, such as a VENTANA DP200 scanner by VENTANA MEDICAL SYSTEMS, Inc. (Tucson, Arizona) or other suitable imaging equipment. Additional imaging devices and systems are described further herein. The skilled artisan will appreciate that the digital color image acquired by the imaging apparatus is conventionally composed of elementary color pixels. Each colored pixel can be coded over three digital components, each comprising the same number of bits, each component corresponding to a primary color, generally red, green, or blue, also denoted by the term "RGB" components.

FIG. 2 provides an overview of the system 200 of the present disclosure and the various modules utilized within the system. In some embodiments, the system 200 employs a computer device or computer-implemented method having one or more processors 209 and one or more memories 201, the one or more memories 201 storing non-transitory computer-readable instructions for execution by the one or more processors to cause the one or more processors to execute certain instructions as described herein.

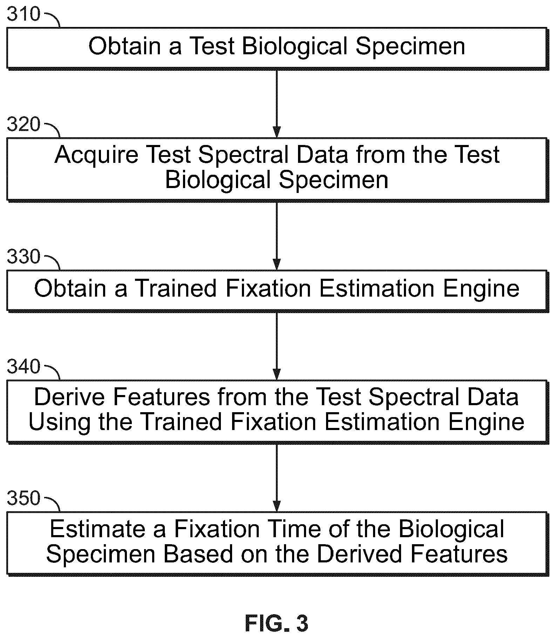

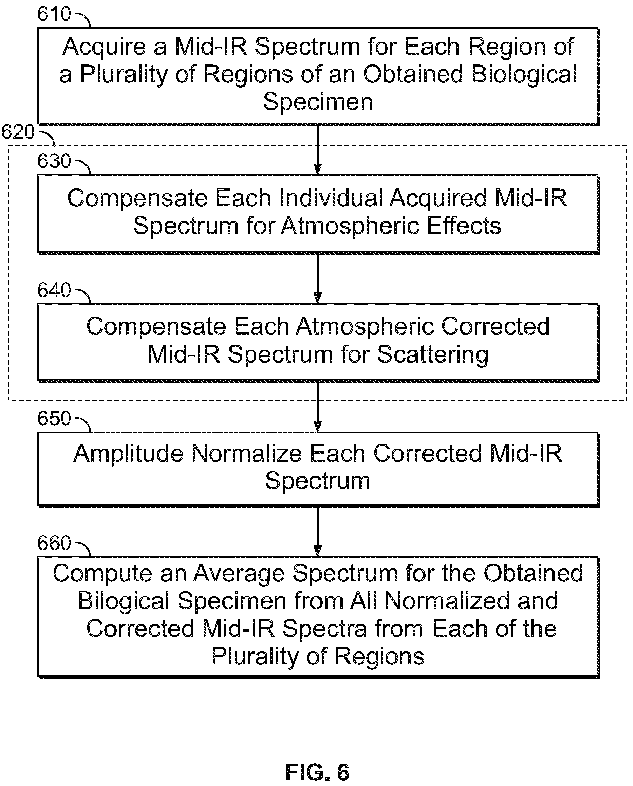

In some embodiments, and as noted above, the system includes a spectral acquisition module 202 for acquiring vibrational spectra, such as mid-IR spectra or RAMAN spectra, of an obtained biological specimen (see, e.g., step 310 of FIG. 3) or any portion thereof (see, e.g., step 320 of FIG. 3). In some embodiments, the system 200 further includes a spectrum processing module 212 adapted to process acquired spectral data. In some embodiments, the spectrum processing module 212 is configured to pre-process spectral data, such as to correct and/or normalize the acquired spectra (see, e.g., steps 620 through 620 of FIG. 6), or to convert acquired transmission spectra to absorption spectra. In other embodiments, the spectrum processing module 212 is configured to average a plurality of acquired spectra derived from a single biological specimen. In yet other embodiments, the spectrum processing module is configured to compute a first derivative or a second derivative of an acquired spectrum.

In some embodiments, the system 200 further includes a training module 211 adapted to receive training spectral data and to use the received training spectral data to train a fixation estimation engine 210 (see, e.g., steps 710 through 730 of FIG. 7). In some embodiments, the system 200 includes a fixation estimation engine 210 which is trained to detect fixation features within test spectral data (see, e.g., step 340 of FIG. 3) and provide an estimate of a fixation duration based on the detected fixation features (see, e.g., step 350 of FIG. 3).

In some embodiments, the trained fixation estimation engine 210 includes one or more machine-learning algorithms. In some embodiments, one or more machine-learning algorithms is based on dimensionality reduction as described further herein. In some embodiments, the dimensionality reduction utilized principal component analysis, such as principal component analysis with discriminate analysis. In other embodiments, the dimensionality reduction is a projection onto latent structure regression. In some embodiments, the unmasking status estimation engine 210 includes a neural network. In other embodiments, the fixation estimation engine includes a supervised classifier. In some embodiments, the fixation estimation engine includes a neural network.

The skilled artisan will also appreciate that additional modules may be incorporated into the workflow or into system 200. In some embodiments, an image acquisition module be run to acquire digital images of a biological specimen or any portion thereof. In other embodiments, an automated algorithm may be run such that cells may be detected, classified, and/or scored (see, e.g., United States Patent Publication No. 2017/0372117).

### SPECTRAL ACOUISITION MODULE AND ACQUIRED SPECTRAL DATA

With reference to FIG. 2, in some embodiments, the system 200 runs a spectral acquisition module 202 to capture vibrational spectra (e.g. using an spectra imaging apparatus 12, such as any of those described above) of at least a portion of a biological specimen. In some embodiments, the biological specimen is unstained, for example it does not include any stains indicative of the presence of a biomarker. In other embodiments, the biological specimen may also include one or more stains, e.g. primary stains, or stains indicative of the presence of one or more biomarkers. Once the spectra are acquired using the spectral acquisition module 202, they may be stored in a storage module 240 (e.g. a local storage module or a networked storage module).

In some embodiments, the vibrational spectra may be acquired from a portion of the biological specimen (and this is regardless of whether the specimen is a training biological specimen or a test biological specimen, as described further herein). In such a case, the spectral acquisition module 202 may be programmed to acquire the vibrational spectra from a predefined portion of the sample, for example by random sampling or by sampling at regular intervals across a grid covering the entire sample. This can also be useful where only specific regions of the sample are relevant for analysis. For example, a region of interest may include a certain type of tissue or a comparatively higher population of a certain type of cell as compared with another region of interest. For example, a region of interest may be selected that includes tonsil tissue but excludes connective tissue. In such a case, the spectral acquisition module 202 may be programmed to collect the vibrational spectra from a predefined portion of a region of interest, for example by random sampling of the region of interest or by sampling at regular intervals across a grid covering the entire region of interest. In embodiments where the sample includes one or more stains, vibrational spectra may be obtained from those regions of interest that do not include any stain or include comparatively less stain than other regions.

In some embodiments, at least two regions of the biological specimen are sampled, and vibrational spectra are acquired for each of the at least two regions (and again, this is regardless of whether the specimen is a training biological specimen or a test biological specimen). In other embodiments, at least 10 regions of the biological specimen are sampled, and vibrational spectra are acquired for each of the at least 10 regions. In yet other embodiments, at least 30 regions of the biological specimen are sampled, and vibrational spectra are acquired for each of the at least 30 regions. In further embodiments, at least 60 regions of the biological specimen are sampled, and vibrational spectra are acquired for each of the at least 60 regions. In yet further embodiments, at least 90 regions of the biological specimen are sampled, and vibrational spectra are acquired for each of the at least 90 regions. In even further embodiments, between about 30 regions and about 150 regions of the biological specimen are sampled, and vibrational spectra are acquired for each of the regions.

In some embodiments, a single vibrational spectrum is acquired per region of the biological specimen. In other embodiments, at least two vibrational spectrum is acquired per region of the biological specimen. In yet other embodiments, at least three vibrational spectra are acquired per region of the biological specimen.

In some embodiments, the acquired spectra or acquired spectral data (used interchangeably herein) which are stored in storage module 240 include "training spectral data." In some embodiments, the training spectral data is derived from training biological specimens, where the training biological specimens may be histological specimens, cytological specimens, or any combination thereof. In some embodiments, the training spectral data are used to train a fixation estimation engine 210, such as through use of the training module 211 as described herein. In some embodiments, the training spectral data includes class labels, such as fixation duration and/or fixation quality.

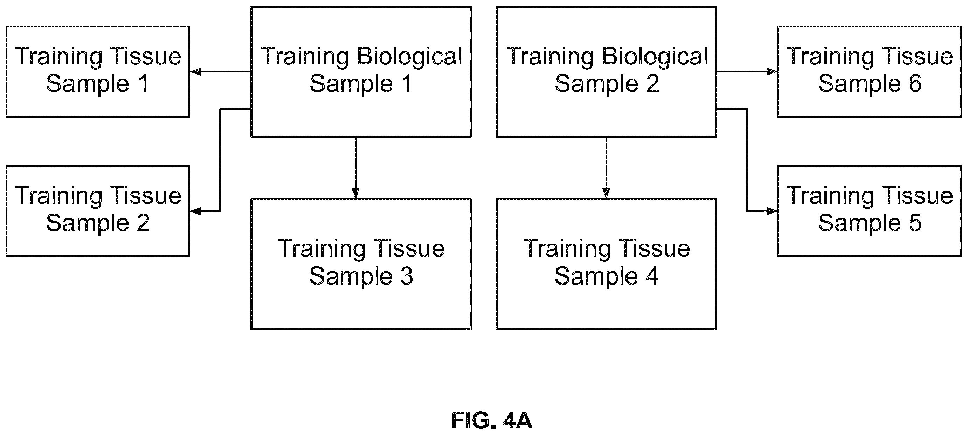

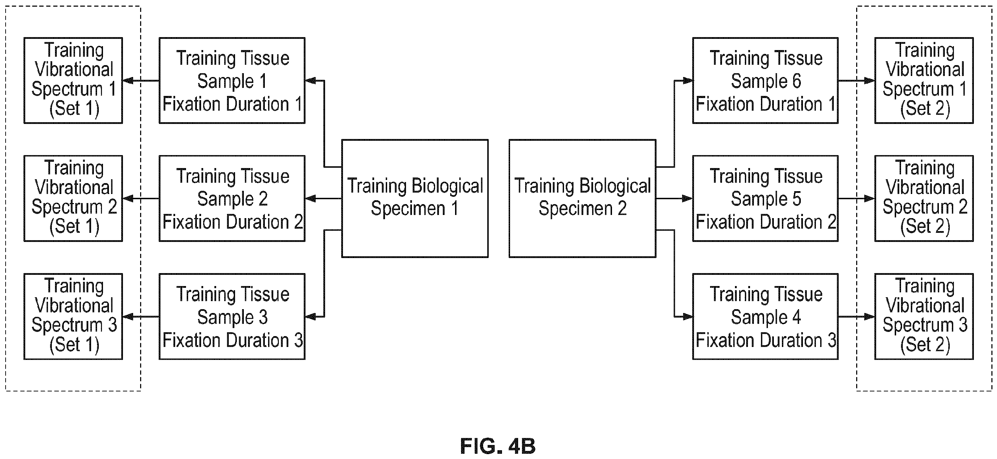

In some embodiments, the training biological specimens are differentially fixed. Differential fixing is a process where a single training biological specimen is divided into a plurality of parts (e.g. a first training tissue sample, a training second tissue sample, and n^{th} training tissue sample) (see, e.g., FIG. 4A), and each part of the plurality of parts is subjected to a different fixation process (see, e.g., FIG. 4B). For example, a single tonsil tissue sample may be divided into 10 or more parts, and each part may be fixed for a pre-determined amount of time. In some embodiments, a sample may sectioned into 3 or more parts, and each part may be fixed for a different amount of time, thus providing three differentially fixed training samples. In other embodiments, a sample may sectioned into 5 or more parts, and each part may be fixed for a different amount of time, thus providing five differentially fixed training samples. In yet other embodiments, a sample may sectioned into 7 or more parts, and each part may be fixed for a different amount of time, thus providing seven differentially fixed training samples. In further embodiments, a sample may sectioned into 9 or more parts, and each part may be fixed for a different amount of time, thus providing nine differentially fixed training samples. This process is illustrated in FIG. 4A. By way of example, a single tonsil tissue sample may be divided into 7 parts, and each part may be differentially fixed for a pre-determined amount of time, e.g. 0 hours, about 1 hour, about 2 hours, about 4 hours, about 6 hours, about 12 hours, about 24 hours, etc.

In some embodiments, any training biological specimen (or portion thereof) may be fixed for any pre-determined amount of time, e.g. about 1 hour, about 2 hours, about 4 hours, about 6 hours, about 12 hours, etc., and the training spectra acquired from the training biological specimens may serve as ground truth in training the fixation estimation engine 210. In this regard, a plurality of training biological specimens may each be partially fixed (for example not treated with fixative for a duration sufficient to seem the sample as "fully fixed" or "adequately fixed"), such as to different degrees, and these partially fixed specimens may be used to train the fixation estimation engine 210 along with training biological specimens which have been determined as being "fully fixed" or "adequately fixed." Additionally, samples which have not be fixed (for example 0 hours of fixation), may also be supplied to the training module 211.

In some embodiments, the training biological specimens which have been at least partially fixed for a pre-determined amount of time are quantitatively verified using functional IHC staining of multiple biomarkers, for example the fixation condition of the specimen has been confirmed by IHC analysis. In some embodiments, each of the training samples is stained for the presence of one or more biomarkers such that functional staining intensity may be evaluated for each training sample. In some embodiments, each training sample is stained for the presence of a single biomarker and then images of the samples are captured using an imaging device and analyzed (such as for staining intensity and/or percent positivity). In other embodiments, each training sample is stained for the presence of two or more biomarkers and then images of the samples are captured using an imaging device and analyzed (such that the staining intensity and/or percent positivity of each of the two or more biomarkers are independently analyzed). For example, different partially fixed specimens may be fixed to different degrees and this different fixation may be verified by staining for the presence of one or more biomarkers (e.g. BLC2, FOXP3, etc.) at different known times (e.g. 6 hours, 12 hours, 24 hours, etc.).

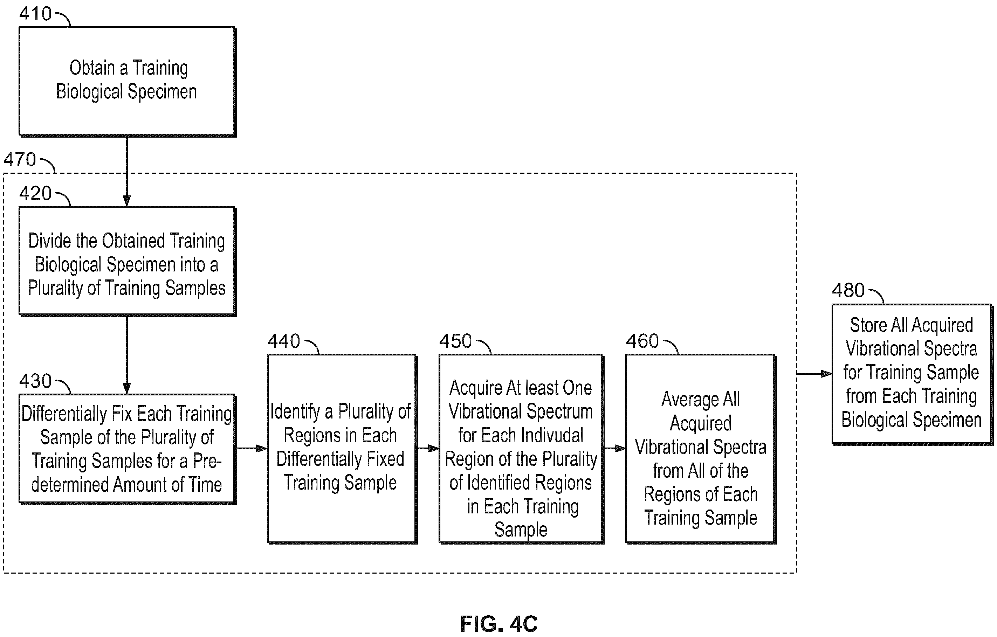

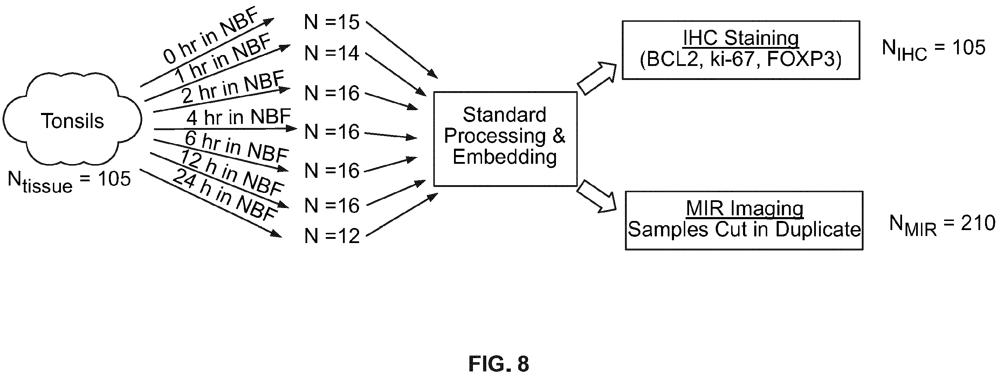

The process of differential fixation and of acquiring spectral data from the differentially fixed samples is further illustrated in FIGS. 4C and 8. As noted above, one or more training biological specimens are first obtained (step 410). Each of the one or more training biological specimens are then divided into at least two parts (step 420). In this way, each of the one or more training biological specimens provide at least two "training samples." Each of these training samples may be differentially fixed, for example each may be fixed for a different pre-determined amount of time (step 430).

Following the differential fixation of the at least two training samples, a plurality of regions in each of the at least two training samples are identified (step 440). Next, at least one vibrational spectrum is acquired for each of the identified regions of the plurality of identified regions (step 450). In some embodiments, the average of each acquired vibrational spectrum from each identified region (or a further processed variant thereof as described further below) is computed to provide an averaged vibrational spectrum for that training sample (step 460). Steps 400 through 460 may be repeated for a plurality of different training biological specimens (see dotted line 470). In some embodiments, the averaged vibrational spectra from all training samples from all training biological specimens (referred to as "training spectral data") are stored (step 480), such as in storage module 240. In this way, the training spectral data may be retrieved from the storage module 240 by the training module 211 for training of a fixation estimation engine 210. In addition to storing the average vibrational spectra from all training samples, the storage module 240 is also adapted to store any class labels associated with the averaged vibrational spectra, e.g. known fixation durations, qualitative fixation estimates, etc.

By way of example, FFPE blocks from a differentially fixed specimens may be sectioned onto vibrational compatible slides. The entire section may then be imaged using a visible low magnification objective in order to coarsely map out the sample on the slide (see, e.g., FIG. 10A). Next, a plurality of spatial regions throughout the sample may be selected to be spectroscopically imaged using spectral acquisition device 12, such as a vibrational microscope (e.g. Bruker Hyperion 3000) using the mercury cadmium telluride detector single point detector (v=900-4000 cm-1, Δv=8 cm-1, averages=16). This is demonstrated in FIGS. 10B and 10C.

The processes described above may be repeated for a plurality of different training biological specimens, where each of the plurality of different training biological specimens may be of the same tissue type or may of a different tissue type. Example 1 herein further describes the methods of preparing training biological specimens and the acquisition of spectral data for use in training a fixation estimation engine 210. Moreover, the processes described above may be repeated for different fixatives reagents or for different fixation processes.

In some embodiments, the acquired spectral data stored in the storage module 240 include "test spectral data." In some embodiments, the test spectral data is derived from test biological specimens, such as specimens derived from a subject (e.g. a human patient), where the test biological specimens may be histological specimens, cytological specimens, or any combination thereof.

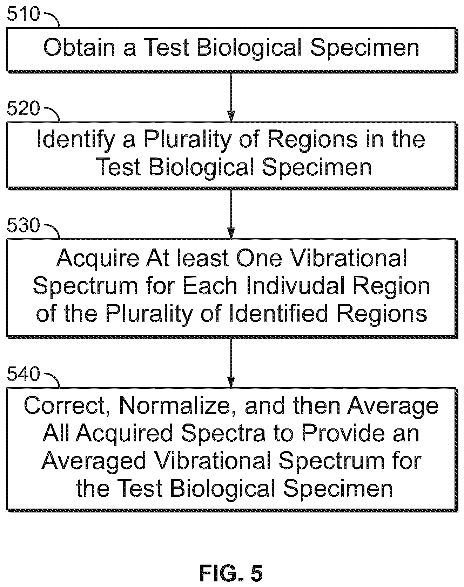

With reference to FIG. 5, a test biological specimen may be obtained (step 510), and then a plurality of spatial regions within the test biological specimen may be identified (step 520). At least one vibrational spectrum may be acquired for each identified region (step 530). The acquired vibrational spectra from all of the regions may then be corrected, normalized, and averaged to provide an averaged vibrational spectrum for the test biological specimen ("test spectral data"). As described further herein, the test spectral data may be supplied to a trained fixation estimation engine 210 such that at least a fixation duration of the test biological specimen may be estimated. The estimated fixation duration may then be used in downstream processes or downstream decision making, e.g. to determine whether a specimen is adequately fixed or not, to determine whether the specimen needs further fixation, or to determine whether the extent of fixation was or is sufficient for a particular IHC or ISH assay.

As noted above, and regardless of whether the spectral data is acquired from a training or test biological specimen, a plurality of vibrational spectra are acquired for each biological specimen, e.g. to account for spatial the spatial heterogeneity of the sample. In some embodiments, the spectral processing module 212 is first utilized to covert each acquired vibrational transmission spectrum to a vibrational absorption spectrum. In some embodiments, transmission spectra and absorbance spectra are directly related via the equation Absorbance = ln(blank transmission / transmission through the tissue) and thus acquired transmission spectra may be converted to absorption spectra.

Once all of the vibrational spectra are converted from transmission to absorbance, in some embodiments, the acquired spectra from all of the various regions are averaged together (such as using a spectral processing module 212), and it is the averaged vibrational spectrum that is used for downstream analysis, for example for training or estimating a fixation duration. In some embodiments, and with reference to FIG. 6, the vibrational spectra acquired from each of the plurality of spatial regions are first normalized and/or corrected prior to their averaging. In some embodiments, vibrational spectra from each region is individually corrected (step 620) to provide a corrected vibrational spectrum. For example, the correction may include compensating each acquired vibrational spectrum for atmospheric effects (step 630) and then compensating each atmospheric corrected vibrational spectrum for scattering (step 640). Next, each corrected vibrational spectrum is amplitude normalized to a pre-determined global maximum (step 650). Subsequently, the collective of the amplitude normalized spectra are averaged (step 660).

This process is demonstrated in FIGS. 10D through 10H. By way of example, raw mid-IR spectra for each point are displayed in FIG. 10D. These spectra were calculated by measuring the transmitted light, across all mid-IR frequencies, and dividing it by the transmission of the slide without tissue to get a measure of how much light the tissue was absorbing. Collected spectra were compensated for atmospheric effects (FIG. 10E) and then baseline corrected to compensate for scattering within the tissue using a concave rubberband correction with 64 baseline points and approximately 8 iterations (FIG. 10F). Each spectrum was then amplitude normalized to a global maximum (FIG. 10G) and then the average spectra from each tissue was calculated by averaging all the spectra from a given tissue together to calculate a high-quality representative spectrum for each sample's slide (FIG. H).

### FIXATION ESTIMATION ENGINE

The systems and methods of the present disclosure employ machine learning techniques to mine spectral data. In the case of a fixation estimation engine in a training mode, the fixation estimation engine may learn features (e.g. fixation features) from a plurality of acquired and processed training spectra and correlate those learned features with class labels associated with the training spectra (e.g. known fixation duration, known functional staining with one or more biomarkers, etc.). In the case of a trained fixation estimation engine (for example a fixation estimation engine that has already been trained using training spectral data and associated class labels), the trained fixation engine may derive features (e.g. fixation features) from a test biological specimen and, based on the learned datasets, predict a fixation status of the test biological specimen based on the derived fixation features.

Machine learning can be generally defined as a type of artificial intelligence (AI) that provides computers with the ability to learn without being explicitly programmed. Machine learning focuses on the development of computer programs that can teach themselves to grow and change when exposed to new data. In other words, machine learning can be defined as the subfield of computer science that gives computers the ability to learn without being explicitly programmed. Machine learning explores the study and construction of algorithms that can learn from and make predictions on data-such algorithms overcome following strictly static program instructions by making data driven predictions or decisions, through building a model from sample inputs. The machine learning described herein may be further performed as described in "Introduction to Statistical Machine Learning," by Sugiyama, Morgan Kaufmann, 2016, 534 pages; "Discriminative, Generative, and Imitative Learning," Jebara, MIT Thesis, 2002, 212 pages; and "Principles of Data Mining (Adaptive Computation and Machine Learning)," Hand et al., MIT Press, 2001, 578 pages. The embodiments described herein may be further configured as described in these references.

In some embodiments, the fixation estimation engine 210 employs "supervised learning" for the task of predicting a fixation state of a test spectrum derived from a test biological specimen. Supervised learning is the machine learning task of learning a function that maps an input to an output based on example input-output pairs. It infers a function from labeled training data (here, the fixation time is the label associated with training spectral data) consisting of a set of training examples (here training spectra). In supervised learning, each example is a pair consisting of an input object (typically a vector) and a desired output value (also called the supervisory signal). A supervised learning algorithm analyzes the training data and produces an inferred function, which can be used for mapping new examples. An optimal scenario will allow for the algorithm to correctly determine the class labels for unseen instances.

The fixation estimation engine 210 may include any type of machine learning algorithm known to those of ordinary skill in the art. Suitable machine learning algorithms include regression algorithms, similarity-based algorithms, feature selection algorithms, regularization method-based algorithms, decision tree algorithms, Bayesian models, kernel-based algorithms (e.g. support vector machines), clustering-based methods, artificial neural networks, deep learning networks, ensemble methods, genetic algorithms, and dimensionality reduction methods. Examples of suitable dimensionality reduction methods include principal component analysis (such as principal component analysis plus discriminant analysis), projection onto latent structure regression, and t-Distributed Stochastic Neighbor Embedding (t-SNE).

In some embodiments, the unmasking status estimation engine 210 utilizes principal component analysis. The main idea of principal component analysis (PCA) is to reduce the dimensionality of a data set consisting of many variables correlated with each other while retaining the variation present in the dataset, up to the maximum extent. The same is done by transforming the variables to a new set of variables, which are known as the principal components (or simply, the PCs) and are orthogonally ordered such that the retention of variation present in the original variables decreases as they move down in the order. In this way, the first principal component retains maximum variation that was present in the original components. The principal components are the eigenvectors of a covariance matrix, and hence they are orthogonal. Principal component analysis and methods of employing the same are described in U.S. Patent Publication No. 2005/0123202 and in U.S. Patent Nos. 6,894,639 and 8,565,488. PCA and Linear Discriminant Analysis are further described by Khan et. al., "Principal Component Analysis-Linear Discriminant Analysis Feature Extractor for Pattern Recognition," "IJCSI International Journal of Computer Sciences Issues, Vol. 8, Issue 6, No. 2, Nov. 2011.

The t-SNE algorithm is a non-linear dimensionality reduction technique well-suited for embedding high-dimensional data for visualization in a low-dimensional space of two or three dimensions. Specifically, it models each high-dimensional object by a two- or three-dimensional point in such a way that similar objects are modeled by nearby points and dissimilar objects are modeled by distant points with high probability. The t-SNE algorithm comprises two main stages. First, t-SNE constructs a probability distribution over pairs of high-dimensional objects in such a way that similar objects have a high probability of being picked while dissimilar points have an extremely small probability of being picked. Second, t-SNE defines a similar probability distribution over the points in the low-dimensional map, and it minimizes the Kullback-Leibler divergence between the two distributions with respect to the locations of the points in the map. The t-SNE algorithm is further described in United States Patent Publication Nos. 2018/0046755, 2014/033 6942 and 2018/0166077.

PLSR is a recent technique that combines features from and generalizes principal component analysis (PCA) and multiple linear regression. Its goal is to predict a set of dependent variables from a set of independent variables or predictors. This prediction is achieved by extracting from the predictors a set of orthogonal factors called latent variables which have the best predictive power. These latent variables can be used to create displays akin to PCA displays. The quality of the prediction obtained from a PLS regression model is evaluated with cross-validation techniques such as the bootstrap and jackknife. There are two main variants of PLS regression: The most common one separates the roles of dependent and independent variables; the second one-gives the same roles to dependent and independent variables. PLSR is further described by Abdi, "Partial Least Squares Regression and Projection on Latent Structure Regression (PLS Regression)," WIREs Computational Statistics, John Wiley & Sons, Inc., 2010.

In some embodiments, the fixation estimation engine 210 utilizes reinforcement learning. Reinforcement Learning (RL) refers to a type of Machine Learning method in which the agent receives a delayed reward in the next time step to evaluate its previous action. Said another way, RL is model-free machine learning paradigm concerned with how software agents ought to take actions in an environment so as to maximize some notion of cumulative reward. Typically, a RL setup is composed of two components, an agent, and an environment. The environment refers to the object that the agent is acting on, while the agent represents the RL algorithm. The environment starts by sending a state to the agent, which then based on its knowledge to take an action in response to that state. After that, the environment sends a pair of next state and reward back to the agent. The agent will update its knowledge with the reward returned by the environment to evaluate its last action. The loop keeps going on until the environment sends a terminal state, which ends to episode. Reinforcement learning algorithms are further described in U.S. Patent Nos. 10,279,474 and 7,395,252.

In some embodiments, the fixation estimation engine 210 includes a Support Vector Machine (" SVM"). In general, an SVM is a classification technique, which is based on statistical learning theory where a nonlinear input data set is converted into a high dimensional linear feature space via kernels for the non-linear case. A support vector machines project a set of training data, E, that represents two different classes into a high-dimensional space by means of a kernel function, K. In this transformed data space, nonlinear data are transformed so that a flat line can be generated (a discriminating hyperplane) to separate the classes so as to maximize the class separation. Testing data are then projected into the high-dimensional space via K, and the test data (such as the features or metrics enumerated below) are classified on the basis of where they fall with respect to the hyperplane. The kernel function K defines the method in which data are projected into the high-dimensional space.

In some embodiments, the fixation estimation engine 210 includes a neural network. In some embodiments, the neural network is configured as a deep learning network. Generally speaking, "deep learning" is a branch of machine learning based on a set of algorithms that attempt to model high level abstractions in data. Deep learning is part of a broader family of machine learning methods based on learning representations of data. An observation can be represented in many ways such as a vector of intensity values per pixel, or in a more abstract way as a set of edges, regions of particular shape, etc. Some representations are better than others at simplifying the learning task. One of the promises of deep learning is replacing handcrafted features with efficient algorithms for unsupervised or semi-supervised feature learning and hierarchical feature extraction.

In some embodiments, the neural network is a generative network. A "generative" network can be generally defined as a model that is probabilistic in nature. In other words, a "generative" network is not one that performs forward simulation or rule-based approaches. Instead, the generative network can be learned (in that its parameters can be learned) based on a suitable set of training data (e.g. a plurality of training spectral data sets). In some embodiments, the neural network is configured as a deep generative network. For example, the network may be configured to have a deep learning architecture in that the network may include multiple layers, which perform a number of algorithms or transformations.

In some embodiments, the neural network includes an autoencoder. An autoencoder neural network is an unsupervised learning algorithm that applies backpropagation (see further description herein), setting the target values to be equal to the inputs. The aim of an autoencoder is to learn a representation (encoding) for a set of data, typically for dimensionality reduction, by training the network to ignore signal "noise." Along with the reduction side, a reconstructing side is learnt, where the autoencoder tries to generate from the reduced encoding a representation as close as possible to its original input. Additional information regarding autoencoders can be found at http://ufldl.stanford.edu/tutorial/unsupervised/Autoencoders/.

In some embodiments, the neural network may be a deep neural network with a set of weights that model the world according to the data that it has been fed to train it. Neural networks typically consist of multiple layers, and the signal path traverses from front to back between the layers. Any neural network may be implemented for this purpose. Suitable neural networks include LeNet, AlexNet, ZFnet, GoogLeNet, VGGNet, VGG16, DenseNet, and the ResNet. In some embodiments, a fully convolutional neural network is utilized, such as described by Long et al., "Fully Convolutional Networks for Semantic Segmentation," Computer Vision and Pattern Recognition (CVPR), 2015 IEEE Conference, June 20015 (INSPEC Accession Number: 15524435).

In some embodiments, the neural network is configured as an AlexNet. For example, the classification network structure can be AlexNet. The term "classification network" is used herein to refer to a CNN, which includes one or more fully connected layers. In general, an AlexNet includes a number of convolutional layers (e.g., 5) followed by a number of fully connected layers (e.g., 3) that are, in combination, configured and trained to classify data.

In other embodiments, the neural network is configured as a GoogleNet. While the GoogleNet architecture may include a relatively high number of layers (especially compared to some other neural networks described herein), some of the layers may be operating in parallel, and groups of layers that function in parallel with each other are generally referred to as inception modules. Other of the layers may operate sequentially. Therefore, a GoogleNet is different from other neural networks described herein in that not all of the layers are arranged in a sequential structure. Examples of neural networks configured as GoogleNets are described in "Going Deeper with Convolutions," by Szegedy et al., CVPR 2015.

In other embodiments, the neural network is configured as a VGG network. For example, the classification network structure can be VGG. VGG networks were created by increasing the number of convolutional layers while fixing other parameters of the architecture. Adding convolutional layers to increase depth is made possible by using substantially small convolutional filters in all of the layers.

In other embodiments, the neural network is configured as a deep residual network. For example, the classification network structure can be a Deep Residual Net or ResNet. Like some other networks described herein, a deep residual network may include convolutional layers followed by fully connected layers, which are, in combination, configured and trained for detection and/or classification. In a deep residual network, the layers are configured to learn residual functions with reference to the layer inputs, instead of learning unreferenced functions. In particular, instead of hoping each few stacked layers directly fit a desired underlying mapping, these layers are explicitly allowed to fit a residual mapping, which is realized by feedforward neural networks with shortcut connections. Shortcut connections are connections that skip one or more layers. A deep residual net may be created by taking a plain neural network structure that includes convolutional layers and inserting shortcut connections which thereby takes the plain neural network and turns it into its residual learning counterpart. Examples of deep residual nets are described in "Deep Residual Learning for Image Recognition" by He et al., NIPS 2015. The neural networks described herein may be further configured as described in this reference.

### TRAINING A FIXATION ESTIMATION ENGINE

In some embodiments, the fixation estimation engine 210 is adapted to operate in a training mode. In some embodiments, a training module 211 is in communication with the fixation estimation engine 210 and is configured to receive training spectral data and supply the training spectral data to the fixation estimation engine 210. In some embodiments, the training module 211 may operate to provide training spectral data to the fixation estimation engine 210 and to operate the fixation estimation engine 210 in its training mode in accordance with any suitable training algorithm, e.g. k-fold cross validation, back propagation, etc. In some embodiments, the training algorithms utilize a known set of training spectral data (such as described herein). In some embodiments, a training module 211 is in communication with the fixation estimation engine 210 and is configured to receive training spectral data (or a further processed variants of the training absorbance spectra data, e.g. a first or second derivative of the training spectral data, magnitudes of individual bands within the training spectra data, the integral of bands within the training spectral data, the ratio of two or more band intensities within the training spectral data, the ratios from second and third order derivatives of the training spectral data, etc.) and supply the training spectral data to the fixation estimation engine 210. In some embodiments, the training module 211 is also adapted to supply the class labels associated with the training spectral data.

In some embodiments, the training algorithms utilize a known set of training spectral data (such as described herein) and a corresponding set of known output class labels (e.g. fixation condition, fixation quality, etc.), and are configured to vary internal connections within the fixation estimation engine 210 such that processing of input training spectral data provides the desired corresponding class labels.

The unmasking status estimation engine 210 may be trained in accordance with any methods known to those of ordinary skill in the art. For example, any of the training methods disclosed in U.S. Patent Publication Nos. 2018/0268255, 2019/0102675, 2015/0356461, 2016/0132786, 2018/0240010, and 2019/0108344.

In some embodiments, the fixation estimation engine 210 is trained using a cross-validation method. Cross-validation is a technique that can be used to aid in model selection and/or parameter tuning when developing a classifier. Cross-validation uses one or more subsets of cases from the set of labeled cases as a test set. For example, in k-fold cross-validation, a set of labeled cases is equally divided into k "folds," for example K-fold cross-validation is a resampling procedure used to evaluate machine learning models. A series of train-then-test cycles is performed, iterating through the k folds such that in each cycle a different fold is used as a test set while the remaining folds are used as the training set. Since each fold is used as the test set at some point, non-randomly selected cases in the set of labeled cases would seemingly bias the cross-validation. For example, in the scenario of 5-fold cross validation (k=5), the data set is split into 5 folds. In the first iteration, the first fold is used to test the model and the rest are used to train the model. In the second iteration, 2nd fold is used as the testing set while the rest serve as the training set. This process is repeated until each fold of the 5 folds have been used as the testing set. Methods of performing k-fold cross validation are further described in US Patent Publication Nos.: 2014/0279734 and 2005/0234753. FIGS. 14A and 14B illustrate the process of training a fixation estimation engine 210 utilizing k-fold cross validation.

In embodiments where the fixation estimation engine 210 includes a neural network, the back propagation algorithm for training the fixation estimation engine 210 is an iterative process in which the connections between network nodes are given some random initial values, and the network is operated to calculate corresponding output vectors for a set of input vectors (the training spectral data set). The output vectors are compared to the desired output of the training spectral data set and the error between the desired and actual output is calculated. The calculated error is propagated back from the output nodes to the input nodes and is used for modifying the values of the network connection weights in order to decrease the error. After each such iteration the training module 211 may calculate a total error for the entire training set and the training module 211 may then repeat the process with another iteration. The training of the fixation estimation engine 210 is complete when the total error reaches a minimum value. If a minimum value of the total error is not reached after a predetermined number of iterations and if the total error is not a constant the training module 211 may consider that the training process does not converge.

In the context of training the fixation estimation engine 210 with acquired spectral data derived from training biological specimens differentially fixed for pre-determined periods of time (described above), each acquired training spectrum is associated with a known fixation duration. In some embodiments, the training spectral data utilized is verified using functional IHC staining of multiple biomarkers, as described above, for example the fixation duration of a training biological specimen is correlated with actual staining data of one or more biomarkers. Indeed, different partially fixed specimens may be fixed to different degrees and this different fixation may be verified by staining for the presence of one or more biomarkers (e.g. BLC2, FOXP3, etc.) at different known times (e.g. about 6 hours, about 12 hours, about 24 hours, etc.). As set forth in the Example herein, biomarker preservation varies depending on the duration of fixation, and even varies once the sample is "fully" fixed, for example fixed for a period of time greater than 6 hours (see also FIGS. 11A - 11D). By way of example, a training spectral data set may be provided, such as to the training module 211, having a known fixation duration of 12 hours, and where this specific training spectral data set was verified through functional IHC staining with one or more biomarkers. By way of further example, additional training spectral data sets may be provided having other known fixation durations, such as 0 hours, 1 hour, 2 hours, 4 hours, 6 hours, etc., where each specific training spectral data set was again verified through functional IHC staining with one or more biomarkers. In this manner, training spectral data is provided which is representative of fixation having a high quality for each of those different pre-determined fixation durations. In some embodiments, the training spectral data sets provided to the training module 211 not only include training absorbance spectra, but also computed first and/or second derivatives of the training absorbance spectra and/or other further processed variants of the training absorbance spectra data.

In some embodiments, acquired training spectral data may also be associated with a "fixation quality." As used herein, a "fixation quality" refers to the extent and/or uniformity of fixation. By way of example, a particular training spectrum provided to the training module 211 may include an associated known fixation duration as verified through functional IHC testing (e.g. 12 hours) and an associated fixation quality (e.g. "good fixation quality," "poor fixation quality," "inadequately fixed," "adequately fixed," etc.). For example, a sample fixed for 0 hours or 1 hour may be associated with a label of "inadequately fixed;" while a sample fixed for 12 hours or 24 hours may be associated with a label of "adequately fixed."

In this way, the fixation estimation engine 210 may be trained not only to detect fixation features associated with a fixation duration, but also a quality of the fixation. For example, training data sets may be provided to pathologists and these training data sets may be annotated by the pathologist to include a qualitative assessment of fixation quality. In this way, a trained fixation estimation engine 210 may not only provide a quantitative estimate of a fixation duration, but also a qualitative assessment of fixation quality. In this regard, it is also possible to train the fixation estimation engine 210 to learn fixation features not only those specimens that have been properly and adequately fixed, but also those that are poorly and inadequately fixed.

When the training of the fixation estimation engine 210 is complete, the system 200 is ready to operate to detect fixation features from test spectral data and then estimate a fixation duration of the test biological specimen based on the detected fixation features. In some embodiments, the fixation estimation engine 210 may be periodically retrained to adapt for variations in input data.

### ESTIMATION OF FIXATION DURATION OF A TEST SPECTRA

Once the fixation estimation engine 210 has been appropriately trained, such as described above, it may be used to detect fixation features within test spectra and, based on the detected fixation features, quantitatively estimate a fixation duration. In some embodiments and depending on how the fixation estimation engine 210 is trained, the trained fixation estimation engine 210 may also provide as an output a qualitative assessment of fixation quality.

In some embodiments, and with reference to FIG. 3, a test biological specimen is obtained (step 310) (such as from a subject suspected of having a certain disease or known to have a certain disease) and then test spectral data is acquired from that test biological specimen (step 320) (see also FIG. 5). In some embodiments, the test spectral data includes the absorbance spectra, the first and/or second derivatives of the absorbance spectra, magnitudes of individual bands within the training spectra data, the integral of bands within the training spectral data, the ratio of two or more band intensities within the training spectral data, the ratios from second and third order derivatives of the training spectral data, etc.

Once test spectral data have been acquired and processed, fixation features may be detected within the test spectral data using the trained fixation estimation engine 210 (step 340). For example, fixation features that may be detected are peak amplitudes, peak positions, peak ratios, a sum of spectral values (such as the integral over a certain spectral range), one or more changes in slope (first derivative) or changes in curvature (second derivative), etc. Based on detected fixation features, an estimate of fixation duration may be computed (step 350). In embodiments where the fixation estimation engine 210 is trained to detect and/or classify a fixation quality, the fixation estimation engine 210 may also provide an estimation of fixation quality.

In some embodiments, the trained fixation estimation engine 210 may also provide as an output an identification of one or more bands and/or a range of wavelengths within acquired spectra that are particularly suitable for use in estimating a fixation duration.

Example 1 herein provides an example of the steps of training a fixation estimation engine, obtaining a test biological specimen, processing the test biological specimen, and using the trained fixation estimation engine in predicting a fixation duration.

### EXAMPLE 1 - PREDICTING FIXATION DURATION USING A TRAINED FIXATION ESTIMATION ENGINE

The Example provided below is illustrative of the methods described herein.

### Introduction

Modern histology is built on the 100+ year old cornerstone technology of formaldehyde fixation which preserves tissue's biostructure by arresting mechanisms of molecular degradation by crosslinking a tissue's biostructure. How thoroughly a sample is fixed can significantly impact detected protein expression and in extreme cases a patient's diagnosis. For example, ASCO/CAP guidelines require samples be fixed for 6 - 72 hours. Currently tissue fixation in clinical practice is laboratory specific and highly variable. Despite its importance, there are however no analytical methods of measuring fixation quality.

Mid infrared spectroscopy (mid-IR) is a powerful optical technique that probes the vibrational state of individual molecules in the tissue and is very sensitive to the conformational state of proteins. This extreme sensitivity makes mid-IR spectroscopy ideally suited for microscopy applications because the presence and even conformational state of endogenous and exogenous materials manifest through changes in the mid-IR absorption profile of the biospecimen. Vibrational spectroscopy has even been used for diagnostic applications, for example to distinguish healthy from cancerous tissue.

Although the exact mechanism of fixation is not fully understood, it is likely a complex synergy of chemistry and conformation changes that occur within the tissue's biostructure. For these reasons, action was taken to investigate if changes in a biospecimen's molecular composition would manifest in changes in the mid-IR spectrum. The ultimate goal was to use the mid-IR spectra, and presumably the fixation signature contained within it, to develop a metrology that could be used to accurately determined the fixation duration and quality of a tissue sample. Such a novel capability would enable the ability to assess the fixation status of a tissue sample with a standardized and objective metrology.

### Methods and Materials

### A. Design of Experiment

In order to definitively study if fixation status could be tracked via mid-IR spectroscopy a large-scale controlled study was designed. One hundred and five individual pieces of tonsil were differentially fixed for either about 0, about 1, about 2, about 4, about 6, about 12, or about 24 hours in room temperature 10% neutral buffered formalin (10% NBF) (see FIG. 8). Between about 12 and about 16 tonsil samples were analyzed within each fixation time. After being fixed in formalin, all tissues were routinely processed by dehydration though increasing concentrations of ethanol, cleared in xylene, and finally embedded in paraffin wax.

Because the samples were all equivalently processed, the only difference between the samples was the amount of time in formalin, for example the chemical fixation time. It is important to note however that all the samples would be considered fixed in ethanol, although fixation in only ethanol is not standard practice in routine histology. In addition to the controlled chemical fixation time, tissues were stained with antibodies to the proteins BCL2, ki-67, and FOXP3. This enabled determination of correlations between fixation time and fixation quality versus functional staining. Biomarker expression was determined quantitatively by analyzing brightfield images with a developed digital quantitation program, as described further herein. To account for intrasample variability in the mid-IR spectra, each paraffin block was cut in duplicate and then all 210 slides were imaged with a mid-IR microscope.

### B. IHC Staining Procedure

Immunohistochemistry assays were performed on a Ventana Discovery XT automated staining instrument according to the manufacturer's instructions. Slides were de-paraffinized using EZ PREP solution (Ventana Medical Systems Inc.) at 90oC and all reagents and incubation times were chosen as directed on package inserts. Slides were developed using the OptiView DAB detection kit (Ventana Medical Systems Inc.) and counterstained with hematoxylin.

### C. Brightfield Imaging and Imaging Processing

Tissue sections were obtained from formalin-fixed paraffin embedded (FFPE) tissues obtained from tissue specimens that were differentially fixed for various amounts of time in about 10% NBF. Four-micron sections were individually stained with 3 antibodies toward BCL2, ki-67 and FOXP3 and developed with DAB stain. All stained slides were then imaged on a Ventana HT Scanner. In order to quantitatively determine expression levels for each stain, an image analysis algorithm was developed that would first segment the tissue on the slide and then would determine regions of the tissue that were not of interest (e.g. connective tissue, stroma). The active regions of the tissue where then analyzed to determine if the tissue was positive or negative for a given protein biomarker. The metric was formalized into the quantitative readout of percent positivity, representing the percent of the tissue's active region that was positive for a given antigen. An overview of the brightfield imaging technique for a whole slide scan is described in FIG. 9A. FIG. 9B shows representative regions of stain for each of the three markers and how the image analysis algorithm classified each 20X field of view.

### D. mid-IR Imaging Acquisition and Data Processing

The same FFPE blocks from the differentially fixed tonsils were sectioned onto mid-IR compatible slides (Kevley Technologies, low-e MirrIR slides). The entire tissue section was then imaged using a visible low magnification objective in order to coarsely map out the sample on the slide (FIG. 10A). Next many regions throughout the sample were selected to be spectroscopically imaged on the mid-IR microscope (e.g. Bruker Hyperion 3000) using the mercury cadmium telluride detector single point detector (v=900-4000 cm-1, Δv=8 cm-1, averages=16). Approximately 100 regions located throughout each tissue were imaged in order to mitigate spatial heterogeneity in the mid-IR spectra (FIGS. 10A - 10C).

Raw mid-IR spectra for each point are displayed in FIG. 10D. These spectra were calculated by measuring the transmitted light, across all mid-IR frequencies, and dividing it by the transmission of the slide without tissue to get a measure of how much light the tissue was absorbing. Collected spectra were compensated for atmospheric effects (FIG. 10E) and then baseline corrected to compensate for scattering within the tissue using a concave rubberband correction with 64 baseline points and approximately 8 iterations (FIG. 10F). Each spectrum was then amplitude normalized to a global maximum (FIG. 10G) and then the average spectra from each tissue was calculated by averaging all the spectra from a given tissue together to calculate a high-quality representative spectrum for each sample's slide (FIG. 10H). Tissue preprocessing was performed in Bruker Optics Opus software.

### Results

### A. Evaluating Fixation Completion with IHC Brightfield Imaging

This section presents quantitative assessment of the three biomarkers expression levels versus fixation time to serve as a gold standard reference to fixation quality. The 12-16 tissue blocks, for each fixation time, were stained for each biomarker and the expression across the whole slide was quantified with an image analysis program as described herein. Summary results in the form of box and whisker plots versus fixation time are displayed in FIGS. 11A - 11C, for BCL2, ki-67, and FOXP3, respectively. BCL2 and FOXP3 were found to be particularly labile and susceptible to improper fixation, as seen by their expression levels steadily increasing monotonically with fixation time. On the other hand, ki-67 was found to be relatively robust to improper fixation as long as the biospecimen was fixed in NBF for at least 1 hour. Finally, these three figures are summarized in FIG. 11D, which displays the average expression level for each biomarker versus fixation time on a scale normalized to the maximum expression at 24 hours for all three biomarkers.

### B. Fixation Time Impact on Amide I mid-IR Band and IHC Results

It is well established in the literature that the Amide I band in the mid-IR spectra is very sensitive not only to the presences of protein absorbers but also to the conformational state of the protein (e.g. Beta-sheet/alpha-Helix/Random Coil). For this reason, the mid-IR absorption of this spectral region was explicitly analyzed looking for deformations that were correlated with the fixation time and therefore fixation quality of the sample. The average mid-IR absorption for all samples for a given fixation time are displayed in FIG. 12A with the approximate location of the Amide I band indicated. The first derivative of each tissue's absorption spectra in the Amide I band was calculated using Savitzky-Golay differentiation to minimize noise in the calculation. The average first derivative plus or minus a standard deviation, as indicated by the shaded regions, is plotted in FIG. 12B.

Interestingly, as fixation time increases there is a clear shift to the right, the amplitude tends to decrease, and the peak tends to widen. These parameters of the band deformation are well characterized by the full width half max (FWHM) and peak location of the band, which is plotted FIG. 12C for all mid-IR slides. In this figure, the samples that are either unfixed (for example 0 hours) or completely fixed (for example 24 hours) are clustered very tightly, indicating a high degree of reproducibility of the Amide I deformation metric, although the other samples fall on more of a spectrum. There is strong correlation between fixation time and Amide I deformation as 0-hour samples cluster in the bottom right and as fixation occurs the peak location shifts toward higher wavenumbers and the FWHM becomes wider. This observation is captured in FIG. 12D, which plots the average shift from the 0hour position for all fixation times. It can be seen that a relatively small shift in the band is observed after about 1 and about 2 hours of fixation and that a large jump is observed at about 4 hours although the band continues to deform as the sample completes fixation all the way out to full fixation at about 24 hours.

Analysis of the primary Amide I band revealed a direct correlation between the fixation time of a biospecimen and the deformation of the Amide I protein band. This discovery was next compared to the IHC expression of three different cancer biomarkers to determine if the Amide I shift was also correlated with functional staining data. To achieve this the average mid-IR shift from the 0-hour sample is plotted versus the percent of each tissue positive for BCL2, ki-67 and FOXP3 in FIGS. 13A - 13C. The results confirm that the deformation in the mid-IR Amide I band is highly correlated with IHC expression. The two markers (BCL2 & FOXP3) that were found to be sensitive to fixation time display highly linear relationships between the Amide I shift and IHC expression. As would therefore be expected, the IHC staining from robust ki-67 staining is not correlated with Amide I deformation because ki-67 staining is not correlated with fixation time/quality whereas Amide I deformation is.

This section confirms that there are biochemical changes in the tissue that occur during formalin fixation that result in a steady and regular deformation of the Amide I band. This finding was confirmed by a fixation time course experiment as well as by comparing mid-IR data to function staining results from fixation dependent biomarkers that closely resemble what the fixation of a sample is in a clinical setting. However, it can also be seen that there is a large spread in the data for partially fixed samples (for example about 1 - about 12 hour of fixation) and that using a metric based solely on shift in the Amide I band the degree of fixation could only be determined very coarsely into improperly fixed samples (Amide I peak locations less than ≈1621cm-1 and Amide I FWHM values less than ≈25cm-1) and properly fixed tissue samples (Amide I peak locations greater than ≈1623cm-1 and Amide I FWHM values greater than ≈35cm-1). Even with this course designation of fixed versus unfixed the accuracy would suffer because samples in between these two regions could not be classified accurately or with great confidence. Additionally, with the Amide I metric it is impossible to distinguish about 4, about 6, about 12, and about 24 hours of NBF fixation. It is known from diagnostic testing that 4 hours is often insufficient fixation time as illustrated by the current HER2 preanalytical guidelines issues by ASCO/CAP that mandate about 6 - about 72 hours of fixation. For these reasons, a more sensitive method of determining fixation time was needed.

### C. Quantitative Machine Learning Model to Predict Fixation Time

In order to develop a more accurate method of determining the fixation time and therefore quality of the tissue biospecimen, a machine learning model was developed based on the projection onto latent structure regression (PLSR) algorithm. In order to train the algorithm, all 210 slides from the differentially fixed blocks were put into a database and 25% (for example 52 tissues) were removed from the dataset to serve as a validation set. The remaining 75% of the samples were used to train a model using 5-fold cross-validation. The final model was tuned by selecting the number of valid components based on the percent of variance explained by each component as well as the mean squared predictive error (MSPE). The final model was then applied to the held out blinded dataset in order to determine the accuracy of the model on blinded tissue spectra. A schematic flowchart of the model development is displayed in FIG. 14B.

The developed model was used to predict the fixation time for all 210 mid-IR tissues, including the training set spectra as well as the blinded holdout spectra. Results are plotted in FIG. 15A. As can be seen from the figure, all predicted fixation times are accurately predicted to be close to their actual or experimental fixation times. Across all fixation times, the model was able to predict the fixation time of blinded tissue within about 1.4 hours. These incredible results indicate that a machine learning model has mined the true molecular fingerprint of formalin fixation and can use it to accurately predict fixation times of unknown samples to on average 1.4 hours. The cumulative distribution functions for the fixation time predictions of the training as well as validation/holdout data are displayed in FIG. 15B. From the figure, it can be seen that the training as well as blinded datasets have nearly identical CDF functions, indicating that the model is not simply overfitting to noise or the underlying structure of the spectra in the training dataset.

One large advantage of this approach is that the algorithm can be used to investigate the molecular fingerprint of fixation, as detected in the mid-IR absorption spectra. The model coefficients are plotted in FIG. 16A, with values significantly greater than zero representing a frequency that is positively correlated with NBF fixation and values below 0 representing wavenumbers that are negatively associated with fixation. As would be expected there is a large contribution from the Amide I band near 1630 cm⁻¹, however several other important bands also contribute to the overall productive power of the algorithm. A number of these bands are potted in FIG. 16B to demonstrate that there are differences in the mid-IR spectra across the wavelength range and even though these differences are impossible for a human to analyze and use to predict fixation time, the developed model is using information throughout the wavelength range to make an accurate assessment of the fixation time.

### Discussion

PCT Publication No. WO/2017/072320 describes a method of evaluating the quality state (such as a fixation status) of a cellular sample. There, a mid-IR spectrum of the sample is obtained, and a classification or quantification algorithm is applied to the MIR spectrum to identify features indicative of the quality state and/or to classify the sample. The quality state may then be used to determine whether the sample is appropriate for an analytical method and/or whether remedial processing (such as further fixation) is appropriate.

PCT Publication No. WO/2017/072320 demonstrated that mid-IR spectroscopy could be generally used to determine if a sample was properly fixed or not fixed by using principal component analysis. This example, and the present disclosure in general, expands on that previous work to verify in a large scale study with over a hundred different pieces of tissue that the actual fixation time of a sample can be accurately determined to on average about 1.4 hours for unknown tissue samples based on a machine learning model of the average mid-IR spectra for individual tissue specimen. This finding was additionally validated with quantitative analysis of functional IHC staining to BCL2, ki-67, and FOXP3 which confirmed the fixation quality of the tissue specimen in this experiment. The present disclosure establishes that an objective and standardized methodology to assess the quality of a tissue samples is possible using mid-IR spectroscopy coupled with an appropriate predictive machine learning models.

Whereas PCT Publication No. WO/2017/072320 disclosed the ability to see differences in the mid-IR spectra based on fixation, the present disclosure work builds a predictive model based on the mining of a plurality of vibrational spectra to predict the actual fixation time with quantitative accuracy. This is an important distinction because PCT Publication No. WO/2017/072320 can primarily only be used to distinguish between unfixed and fully fixed tissue (for example about 0-hour versus about 24 hours). As more tissue diagnostic assays are developed on increasingly labile biomolecules (RNA, methylation states, phosphorylation, etc.), it is envisioned that even more strict quality assurance measures related to fixation and tissue quality will be needed to ensure accurate diagnosis. The systems and methods of the present disclosure addresses this currently unmet future diagnostic need.

It is believed that this can be clearly illustrated by looking at the HER2 ASCO/CAP guidelines of fixation which require at least about 6 hours of fixation. The present method is thus significantly more accurate at predicting fixation durations and is quantitative versus qualitative in nature which offers another significant benefit. Additionally, this is a much stronger assertion that the mid-IR signal, when coupled with the appropriate predictive algorithms, can accurately predict fixation durations because the fixation estimation engine was trained on a large number of tissue samples. Indeed, the fixation estimation engine was trained on quantitatively verified data, for example using functional IHC staining of multiple biomarkers. The trained fixation estimation engine was verified to work on blinded biological specimens that the trained fixation estimation engine never saw before to act as a validation training set. The systems and methods disclosed here would, it is believed, make implementing this technology easier, more practical, and cheaper because the wavelengths of interests can readily be determined from the used algorithm. This capability enables the accurate identification of important wavelengths so that only those wavelengths are included in the calculation to speed up computation time. The systems and methods of the present disclosure may also enable deeper study in the complicated and currently mysterious chemical processes that occur within a tissue specimen during formalin fixation.

### EXAMPLE 2 - TRAINING A FIXATION ESTIMATION ENGINE

In one embodiment, a projection-onto-latent-structure (also partial least squares regression) PLSR model is trained using spectra acquired from a mid-IR microscope. The mid-IR spectra is collected at roughly one hundred locations throughout the tissue, so the average spectrum is representative of the average of the tissue. All spectra were atmospherically corrected to remove CO₂ contamination, baseline-corrected using concave rubber band correction with 10 iterations and 64 baseline points, then spectra were amplitude normalized. Finally, all the spectra from each tissue were averaged together. Next a PLSR regression model was trained by splitting the data into 5 parts, meaning every 5th spectrum was pulled out of the data set and only used exclusively for validation of the model. About 80% of the tissue's spectra was then used to train a PLSR model using 2-fold cross validation and the raw absorption spectra (as opposed to the 1^{st}, 2^{nd}, etc. derivative spectra). In some models the region of spectra between about 2750 to about 2800 and between about 3700 to about 4000 was not cropped out, although in other model, in particular with derivative spectra that region can be cropped out or set to 0.

An initial model was trained by analyzing the training set and determining which spectra features correlate with the known fixation durations of each respective tissue. The accuracy of the training set was then interrogated by using the developed model to back project the fixation durations of the known samples. Next, the model had was calibrated to obtain a generalizable model that would work for unknown or blinded tissues. This was done by analyzing the mean-squared-prediction-error (MSPE) of the model the was calculated during the cross-validation of the training set and determining how many components are needed to accurately characterize the true fixation signature of the spectra. Additionally, the percent of variance explained in the response variable (e.g. 'Y' or fixation duration in this example) and the predictor variable (e.g. absorption by wavelength) were plotted versus number of components in the model (see FIGS. 17A and 17B).

Once the proper number of components was selected, the model was retrained and evaluated on both the training set as well as the blinded holdout spectra. In this example, a model was built using the first 20 components and that performance was evaluated. This was done by analyzing the error (absolute deviation of experimental fixation duration versus model-predicted fixation duration) in fixation durations for the training and validation training set. The cumulative distribution function (CDF) for the training and validation were analyzed and should be near equivalent, in order to make sure the model was as accurate as possible as well as generalizable to blinded data. The CDF indicates the model was well trained because the error was identical for training as well as predicted spectra (validation) spectra (see FIG. 17C). It could also be seen from the box and whisker plot (see FIG. 17D), that the number of outlier is very low, another critical component of a well-trained model. Due to the numerous variable involved in training a model, the process of training the model is inherently iterative as tweaking any variable or process along the training path will impact the accuracy of the model.

### Other System Components

The system 200 of the present disclosure may be tied to a specimen processing apparatus that can perform one or more preparation processes on the tissue specimen. The preparation process can include, without limitation, deparaffinizing a specimen, conditioning a specimen (e.g., cell conditioning), staining a specimen, performing antigen retrieval, performing immunohistochemistry staining (including labeling) or other reactions, and/or performing in situ hybridization (e.g., SISH, FISH, etc.) staining (including labeling) or other reactions, as well as other processes for preparing specimens for microscopy, microanalyses, mass spectrometric methods, or other analytical methods.

The processing apparatus can apply fixatives to the specimen. Fixatives can include cross-linking agents (such as aldehydes, e.g., formaldehyde, paraformaldehyde, and glutaraldehyde, as well as non-aldehyde cross-linking agents), oxidizing agents (e.g., metallic ions and complexes, such as osmium tetroxide and chromic acid), protein-denaturing agents (e.g., acetic acid, methanol, and ethanol), fixatives of unknown mechanism (e.g., mercuric chloride, acetone, and picric acid), combination reagents (e.g., Carnoy's fixative, methacarn, Bouin's fluid, B5 fixative, Rossman's fluid, and Gendre's fluid), microwaves, and miscellaneous fixatives (e.g., excluded volume fixation and vapor fixation).

If the specimen is a sample embedded in paraffin, the sample can be deparaffinized using appropriate deparaffinizing fluid(s). After the paraffin is removed, any number of substances can be successively applied to the specimen. The substances can be for pretreatment (e.g., to reverse protein-crosslinking, expose cells acids, etc.), denaturation, hybridization, washing (e.g., stringency wash), detection (e.g., link a visual or marker molecule to a probe), amplifying (e.g., amplifying proteins, genes, etc.), counterstaining, coverslipping, or the like.

The specimen processing apparatus can apply a wide range of substances to the specimen. The substances include, without limitation, stains, probes, reagents, rinses, and/or conditioners. The substances can be fluids (e.g., gases, liquids, or gas/liquid mixtures), or the like. The fluids can be solvents (e.g., polar solvents, non-polar solvents, etc.), solutions (e.g., aqueous solutions or other types of solutions), or the like. Reagents can include, without limitation, stains, wetting agents, antibodies (e.g., monoclonal antibodies, polyclonal antibodies, etc.), antigen recovering fluids (e.g., aqueous- or non-aqueous-based antigen retrieval solutions, antigen recovering buffers, etc.), or the like. Probes can be an isolated cells acid or an isolated synthetic oligonucleotide, attached to a detectable label or reporter molecule. Labels can include radioactive isotopes, enzyme substrates, cofactors, ligands, chemiluminescent or fluorescent agents, haptens, and enzymes. As used herein, the term "fluid" refers to any liquid or liquid composition, including water, solvents, buffers, solutions (e.g. polar solvents, non-polar solvents), and/or mixtures. The fluid may be aqueous or non-aqueous. Non-limiting examples of fluids include washing solutions, rinsing solutions, acidic solutions, alkaline solutions, transfer solutions, and hydrocarbons (e.g., alkanes, isoalkanes and aromatic compounds such as xylene). In some embodiments, washing solutions include a surfactant to facilitate spreading of the washing liquids over the specimen-bearing surfaces of the slides. In some embodiments, acid solutions include deionized water, an acid (e.g., acetic acid), and a solvent. In some embodiments, alkaline solutions include deionized water, a base, and a solvent. In some embodiments, transfer solutions include one or more glycol ethers, such as one or more propylene-based glycol ethers (e.g., propylene glycol ethers, di(propylene glycol) ethers, and tri(propylene glycol) ethers, ethylene-based glycol ethers (e.g., ethylene glycol ethers, di(ethylene glycol) ethers, and tri(ethylene glycol) ethers), and functional analogs thereof. Non-liming examples of buffers include citric acid, potassium dihydrogen phosphate, boric acid, diethyl barbituric acid, piperazine-N,N'-bis(2-ethanesulfonic acid), dimethylarsinic acid, 2-(N-morpholino)ethanesulfonic acid, tris(hydroxymethyl)methylamine (TRIS), 2-(N-morpholino)ethanesulfonic acid (TAPS), N,N-bis(2-hydroxyethyl)glycine(Bicine), N-tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), and combinations thereof. In some embodiments, the unmasking agent is water. In other embodiments, the buffer may be comprised of tris(hydroxymethyl)methylamine (TRIS), 2-(N-morpholino)ethanesulfonic acid (TAPS), N,N-bis(2-hydroxyethyl)glycine(Bicine), N -tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), or a combination thereof. Additional wash solutions, transfer solutions, acid solutions, and alkaline solutions are described in United States Patent Application Publication No. 2016/0282374.

Staining may performed with a histochemical staining module or separate platform, such as an automated IHC/ISH slide stainer. Automated IHC/ISH slide stainers typically include at least: reservoirs of the various reagents used in the staining protocols, a reagent dispense unit in fluid communication with the reservoirs for dispensing reagent to onto a slide, a waste removal system for removing used reagents and other waste from the slide, and a control system that coordinates the actions of the reagent dispense unit and waste removal system. In addition to performing staining steps, many automated slide stainers can also perform steps ancillary to staining (or are compatible with separate systems that perform such ancillary steps), including: slide baking (for adhering the sample to the slide), dewaxing (also referred to as deparaffinization), antigen retrieval, counterstaining, dehydration and clearing, and coverslipping. Prichard, Overview of Automated Immunohistochemistry, Arch Pathol Lab Med., Vol. 138, pp. 1578-1582 (2014), describes several specific examples of automated IHC/ISH slide stainers and their various features, including the intelliPATH (Biocare Medical), WAVE (Celerus Diagnostics), DAKO OMNIS and DAKO AUTOSTAINER LINK 48 (Agilent Technologies), BENCHMARK (Ventana Medical Systems, Inc.), Leica BOND, and Lab Vision Autostainer (Thermo Scientific) automated slide stainers. Additionally, Ventana Medical Systems, Inc. is the assignee of a number of United States patents disclosing systems and methods for performing automated analyses, including U.S. Pat. Nos. 5,650,327, 5,654,200, 6,296,809, 6,352,861, 6,827,901 and 6,943,029, and U.S. Published Patent Application Nos. 20030211630 and 20040052685. As used herein, the term "reagent" refers to solutions or suspensions including one or more agents capable of covalently or non-covalently reacting with, coupling with, interacting with, or hybridizing to another entity. Non-limiting examples of such agents include specific-binding entities, antibodies (primary antibodies, secondary antibodies, or antibody conjugates), nucleic acid probes, oligonucleotide sequences, detection probes, chemical moieties bearing a reactive functional group or a protected functional group, enzymes, solutions or suspensions of dye or stain molecules.

Commercially-available staining units typically operate on one of the following principles: (1) open individual slide staining, in which slides are positioned horizontally and reagents are dispensed as a puddle on the surface of the slide containing a tissue sample (such as implemented on the DAKO AUTOSTAINER Link 48 (Agilent Technologies) and intelliPATH (Biocare Medical) stainers); (2) liquid overlay technology, in which reagents are either covered with or dispensed through an inert fluid layer deposited over the sample (such as implemented on VENTANA BenchMark and DISCOVERY stainers); (3) capillary gap staining, in which the slide surface is placed in proximity to another surface (which may be another slide or a coverplate) to create a narrow gap, through which capillary forces draw up and keep liquid reagents in contact with the samples (such as the staining principles used by DAKO TECHMATE, Leica BOND, and DAKO OMNIS stainers). Some iterations of capillary gap staining do not mix the fluids in the gap (such as on the DAKO TECHMATE and the Leica BOND). In variations of capillary gap staining termed dynamic gap staining, capillary forces are used to apply sample to the slide, and then the parallel surfaces are translated relative to one another to agitate the reagents during incubation to effect reagent mixing (such as the staining principles implemented on DAKO OMNIS slide stainers (Agilent)). In translating gap staining, a translatable head is positioned over the slide. A lower surface of the head is spaced apart from the slide by a first gap sufficiently small to allow a meniscus of liquid to form from liquid on the slide during translation of the slide. A mixing extension having a lateral dimension less than the width of a slide extends from the lower surface of the translatable head to define a second gap smaller than the first gap between the mixing extension and the slide. During translation of the head, the lateral dimension of the mixing extension is sufficient to generate lateral movement in the liquid on the slide in a direction generally extending from the second gap to the first gap. See WO 2011-139978 A1. It has recently been proposed to use inkjet technology to deposit reagents on slides. See WO 2016-170008 A1. This list of staining technologies is not intended to be comprehensive, and any fully or semi-automated system for performing biomarker staining may be incorporated into the histochemical staining platform.

Where a morphologically-stained sample is also desired, an automated H&E staining platform may be used. Automated systems for performing H&E staining typically operate on one of two staining principles: batch staining (also referred to as "dip 'n dunk") or individual slide staining. Batch stainers generally use vats or baths of reagents in which many slides are immersed at the same time. Individual slide stainers, on the other hand, apply reagent directly to each slide, and no two slides share the same aliquot of reagent. Examples of commercially available H&E stainers include the VENTANA SYMPHONY (individual slide stainer) and VENTANA HE 600 (individual slide stainer) series H&E stainers from Roche; the Dako CoverStainer (batch stainer) from Agilent Technologies; the Leica ST4020 Small Linear Stainer (batch stainer), Leica ST5020 Multistainer (batch stainer), and the Leica ST5010 Autostainer XL series (batch stainer) H&E stainers from Leica Biosystems Nussloch GmbH.

After the specimens are stained, the stained samples can be manually analyzed on a microscope, and/or digital images of the stained samples can be acquired for archiving and/or digital analysis. Digital images can be captured via a scanning platform such as a slide scanner that can scan the stained slides at 20x, 40x, or other magnifications to produce high resolution whole-slide digital images. At a basic level, the typical slide scanner includes at least: (1) a microscope with lens objectives, (2) a light source (such as halogen, light emitting diode, white light, and/or multispectral light sources, depending on the dye), (3) robotics to move glass slides around or to move the optics around the slide or both, (4) one or more digital cameras for image capture, (5) a computer and associated software to control the robotics and to manipulate, manage, and view digital slides. Digital data at a number of different X-Y locations (and in some cases, at multiple Z planes) on the slide are captured by the camera's charge-coupled device (CCD), and the images are joined together to form a composite image of the entire scanned surface. Common methods to accomplish this include:
(1) Tile based scanning, in which the slide stage or the optics are moved in very small increments to capture square image frames, which overlap adjacent squares to a slight degree. The captured squares are then automatically matched to one another to build the composite image; and
(2) Line-based scanning, in which the slide stage moves in a single axis during acquisition to capture a number of composite image "strips." The image strips can then be matched with one another to form the larger composite image.

A detailed overview of various scanners (both fluorescent and brightfield) can be found at Farahani et al., Whole slide imaging in pathology: advantages, limitations, and emerging perspectives, Pathology and Laboratory Medicine Int'l, Vol. 7, p. 23-33 (June 2015). Examples of commercially available slide scanners include: 3DHistech PANNORAMIC SCAN II; DigiPath PATHSCOPE; Hamamatsu NANOZOOMER RS, HT, and XR; Huron TISSUESCOPE 4000, 4000XT, and HS; Leica SCANSCOPE AT, AT2, CS, FL, and SCN400; Mikroscan D2; Olympus VS120-SL; Omnyx VL4, and VL120; PerkinElmer LAMINA; Philips ULTRA-FAST SCANNER; Sakura Finetek VISIONTEK; Unic PRECICE 500, and PRECICE 600x; VENTANA ISCAN COREO and ISCAN HT; and Zeiss AXIO SCAN.Z1. Other exemplary systems and features can be found in, for example, WO2011-049608) or in U.S. Patent Application No. 61/533,114, filed on Sep. 9, 2011, entitled IMAGING SYSTEMS, CASSETTES, AND METHODS OF USING THE SAME.

In some embodiments, any imaging may be accomplished using any of the systems disclosed in U.S. Patent Nos. 10,317,666 and 10,313,606. The imaging apparatus may be a brightfield imager such as the iScan Coreo^{™} brightfield scanner or the DP200 scanner sold by Ventana Medical Systems, Inc.

In some cases, the images may be analyzed on an image analysis system. Image analysis system may include one or more computing devices such as desktop computers, laptop computers, tablets, smartphones, servers, application-specific computing devices, or any other type(s) of electronic device(s) capable of performing the techniques and operations described herein. In some embodiments, image analysis system may be implemented as a single device. In other embodiments, image analysis system may be implemented as a combination of two or more devices together achieving the various functionalities discussed herein. For example, image analysis system may include one or more server computers and a one or more client computers communicatively coupled to each other via one or more local-area networks and/or wide-area networks such as the Internet. The image analysis system typically includes at least a memory, a processor, and a display. Memory may include any combination of any type of volatile or non-volatile memories, such as random-access memories (RAMs), read-only memories such as an Electrically-Erasable Programmable Read-Only Memory (EEPROM), flash memories, hard drives, solid state drives, optical discs, and the like. It is appreciated that memory can be included in a single device and can also be distributed across two or more devices. Processor may include one or more processors of any type, such as central processing units (CPUs), graphics processing units (GPUs), special-purpose signal or image processors, field-programmable gate arrays (FPGAs), tensor processing units (TPUs), and so forth. It is appreciated that processor can be included in a single device and can also be distributed across two or more devices. Display may be implemented using any suitable technology, such as LCD, LED, OLED, TFT, Plasma, etc. In some implementations, display may be a touch-sensitive display (a touchscreen). Image analysis system also typically includes a software system stored on the memory comprising a set of instructions implementable on the processor, the instructions comprising various image analysis tasks, such as object identification, stain intensity quantification, and the like. Exemplary commercially-available software packages useful in implementing modules as disclosed herein include VENTANA VIRTUOSO; Definiens TISSUE STUDIO, DEVELOPER XD, and IMAGE MINER; and Visopharm BIOTOPIX, ONCOTOPIX, and STEREOTOPIX software packages.

After the specimens are processed, a user can transport specimen-bearing slides to the imaging apparatus. In some embodiments, the imaging apparatus is a brightfield imager slide scanner. One brightfield imager is the iScan Coreo brightfield scanner sold by Ventana Medical Systems, Inc. In automated embodiments, the imaging apparatus is a digital pathology device as disclosed in International Patent Application No.: PCT/US2010/002772 (Patent Publication No.: WO/2011/049608) entitled IMAGING SYSTEM AND TECHNIQUES or disclosed in U.S. Patent Application No. 61/533,114, filed on Sep. 9, 2011, entitled IMAGING SYSTEMS, CASSETTES, AND METHODS OF USING THE SAME. International Patent Application No. PCT/US2010/002772 and U.S. Patent Application No. 61/533,114.

Embodiments of the subject matter and the operations described in this specification can be implemented in digital electronic circuitry, or in computer software, firmware, or hardware, including the structures disclosed in this specification and their structural equivalents, or in combinations of one or more of them. Embodiments of the subject matter described in this specification can be implemented as one or more computer programs, for example, one or more modules of computer program instructions, encoded on computer storage medium for execution by, or to control the operation of, data processing apparatus. Any of the modules described herein may include logic that is executed by the processor(s). "Logic," as used herein, refers to any information having the form of instruction signals and/or data that may be applied to affect the operation of a processor. Software is an example of logic.

A computer storage medium can be, or can be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or can be included in, one or more separate physical components or media (e.g., multiple CDs, disks, or other storage devices). The operations described in this specification can be implemented as operations performed by a data processing apparatus on data stored on one or more computer-readable storage devices or received from other sources.

The term "programmed processor" encompasses all kinds of apparatus, devices, and machines for processing data, including by way of example a programmable microprocessor, a computer, a system on a chip, or multiple ones, or combinations, of the foregoing. The apparatus can include special purpose logic circuitry, e.g., an FPGA (field programmable gate array) or an ASIC (application-specific integrated circuit). The apparatus also can include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing, and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, subprograms, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random-access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

To provide for interaction with a user, embodiments of the subject matter described in this specification can be implemented on a computer having a display device, e.g., an LCD (liquid crystal display), LED (light emitting diode) display, or OLED (organic light emitting diode) display, for displaying information to the user and a keyboard and a pointing device, e.g., a mouse or a trackball, by which the user can provide input to the computer. In some implementations, a touch screen can be used to display information and receive input from a user. Other kinds of devices can be used to provide for interaction with a user as well; for example, feedback provided to the user can be in any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks). For example, the network 20 of FIG. 1 can include one or more local area networks.

The computing system can include any number of clients and servers. A client and server are generally remote from each other and typically interact through a communication network. The relationship of client and server arises by virtue of computer programs running on the respective computers and having a client-server relationship to each other. In some embodiments, a server transmits data (e.g., an HTML page) to a client device (e.g., for purposes of displaying data to and receiving user input from a user interacting with the client device). Data generated at the client device (e.g., a result of the user interaction) can be received from the client device at the server.

### Examples of Biomarkers

As noted herein, the fixation estimation engine is trained using training spectral data sets acquired from a plurality of differentially fixed training biological specimens. In some embodiments, the class labels of known fixation duration are verified through functional IHC testing. Identified below are non-limiting examples of biomarkers whose expression may be determined through functional IHC staining. Certain markers are characteristic of particular cells, while other markers have been identified as being associated with a particular disease or condition. Examples of known prognostic markers include enzymatic markers such as, for example, galactosyl transferase II, neuron specific enolase, proton ATPase-2, and acid phosphatase. Hormone or hormone receptor markers include human chorionic gonadotropin (HCG), adrenocorticotropic hormone, carcinoembryonic antigen (CEA), prostate-specific antigen (PSA), estrogen receptor, progesterone receptor, androgen receptor, gC1q-R/p33 complement receptor, IL-2 receptor, p75 neurotrophin receptor, PTH receptor, thyroid hormone receptor, and insulin receptor.

Lymphoid markers include alpha-1-antichymotrypsin, alpha-1-antitrypsin, B cell marker, bcl-2, bcl-6, B lymphocyte antigen 36 kD, BM1 (myeloid marker), BM2 (myeloid marker), galectin-3, granzyme B, HLA class I Antigen, HLA class II (DP) antigen, HLA class II (DQ) antigen, HLA class II (DR) antigen, human neutrophil defensins, immunoglobulin A, immunoglobulin D, immunoglobulin G, immunoglobulin M, kappa light chain, kappa light chain, lambda light chain, lymphocyte/histocyte antigen, macrophage marker, muramidase (lysozyme), p80 anaplastic lymphoma kinase, plasma cell marker, secretory leukocyte protease inhibitor, T cell antigen receptor (JOVI 1), T cell antigen receptor (JOVI 3), terminal deoxynucleotidyl transferase, unclustered B cell marker.

Tumor markers include alpha fetoprotein, apolipoprotein D, BAG-1 (RAP46 protein), CA19-9 (sialyl lewisa), CA50 (carcinoma associated mucin antigen), CA125 (ovarian cancer antigen), CA242 (tumor associated mucin antigen), chromogranin A, clusterin (apolipoprotein J), epithelial membrane antigen, epithelial-related antigen, epithelial specific antigen, epidermal growth factor receptor, estrogen receptor (ER), gross cystic disease fluid protein-15, hepatocyte specific antigen, HER2, heregulin, human gastric mucin, human milk fat globule, MAGE-1, matrix metalloproteinases, melan A, melanoma marker (HMB45), mesothelin, metallothionein, microphthalmia transcription factor (MITF), Muc-1 core glycoprotein. Muc-1 glycoprotein, Muc-2 glycoprotein, Muc-5AC glycoprotein, Muc-6 glycoprotein, myeloperoxidase, Myf-3 (Rhabdomyosarcoma marker), Myf-4 (Rhabdomyosarcoma marker), MyoD1 (Rhabdomyosarcoma marker), myoglobin, nm23 protein, placental alkaline phosphatase, prealbumin, progesterone receptor, prostate specific antigen, prostatic acid phosphatase, prostatic inhibin peptide, PTEN, renal cell carcinoma marker, small intestinal mucinous antigen, tetranectin, thyroid transcription factor-1, tissue inhibitor of matrix metalloproteinase 1, tissue inhibitor of matrix metalloproteinase 2, tyrosinase, tyrosinase-related protein-1, villin, von Willebrand factor, CD34, CD34, Class II, CD51 Ab-1, CD63, CD69, Chk1, Chk2, claspin C-met, COX6C, CREB, Cyclin D1, Cytokeratin, Cytokeratin 8, DAPI, Desmin, DHP (1-6 Diphenyl-1,3,5-Hexatriene), E-Cadherin, EEA1, EGFR, EGFRvIII, EMA (Epithelial Membrane Antigen), ER, ERB3, ERCC1, ERK, E-Selectin, FAK, Fibronectin, FOXP3, Gamma-H2AX, GB3, GFAP, Giantin, GM130, Golgin 97, GRB2, GRP78BiP, GSK3 Beta, HER-2, Histone 3, Histone 3_K14-Ace [Anti-acetyl-Histone H3 (Lys 14)], Histone 3_K18-Ace [Histone H3-Acetyl Lys 18), Histone 3_K27-TriMe, [Histone H3 (trimethyl K27)], Histone 3_K4-diMe [Anti-dimethyl-Histone H3 (Lys 4)], Histone 3_K9-Ace [Acetyl-Histone H3 (Lys 9)], Histone 3_K9-triMe [Histone 3-trimethyl Lys 9], Histone 3_S10-Phos [Anti-Phospho Histone H3 (Ser 10), Mitosis Marker], Histone 4, Histone H2A.X-5139-Phos [Phospho Histone H2A.X (Ser139)antibody], Histone H2B, Histone H3_DiMethyl K4, Histone H4_TriMethyl K20-Chip grad, HSP70, Urokinase, VEGF R1, ICAM-1, IGF-1, IGF-1R, IGF-1 Receptor Beta, IGF-II, IGF-IIR, IKB-Alpha IKKE, IL6, IL8, Integrin alpha V beta 3, Integrin alpha V beta6, Integrin Alpha V/CD51, integrin B5, integrin B6, Integrin B8, Integrin Beta 1(CD 29), Integrin beta 3, Integrin beta 5 integrinB6, IRS-1, Jagged 1, Anti-protein kinase C Beta2, LAMP-1, Light Chain Ab-4 (Cocktail), Lambda Light Chain, kappa light chain, M6P, Mach 2, MAPKAPK-2, MEK 1, MEK 1/2 (Ps222), MEK 2, MEK1/2 (47E6), MEK1/2 Blocking Peptide, MET/HGFR, MGMT, Mitochondrial Antigen, Mitotracker Green F M, MMP-2, MMP9, E-cadherin, mTOR, ATPase, N-Cadherin, Nephrin, NFKB, NFKB p105/p50, NF-KB P65, Notch 1, Notch 2, Notch 3, OxPhos Complex IV, p130Cas, p38 MAPK, p44/42 MAPK antibody, P504S, P53, P70, P70 S6K, Pan Cadherin, Paxillin, P-Cadherin, PDI, pEGFR, Phospho AKT, Phospho CREB, phospho EGF Receptor, Phospho GSK3 Beta, Phospho H3, Phospho HSP-70, Phospho MAPKAPK-2, Phospho MEK1/2, phospho p38 MAP Kinase, Phospho p44/42 MAPK, Phospho p53, Phospho PKC, Phospho S6 Ribosomal Protein, Phospho Src, phospho-Akt, Phospho-Bad, Phospho-IKB-a, phospho-mTOR, Phospho-NF-kappaB p65, Phospho-p38, Phospho-p44/42 MAPK, Phospho-p70 S6 Kinase, Phospho-Rb, phospho-Smad2, PIM1, PIM2, PKC β, Podocalyxin, PR, PTEN, R1, Rb 4H1, R-Cadherin, ribonucleotide Reductase, RRM1, RRM11, SLC7A5, NDRG, HTF9C, HTF9C, CEACAM, p33, S6 Ribosomal Protein, Src, Survivin, Synapopodin, Syndecan 4, Talin, Tensin, Thymidylate Synthase, Tuberlin, VCAM-1, VEGF, Vimentin, Agglutinin, YES, ZAP-70 and ZEB.

Cell cycle associated markers include apoptosis protease activating factor-1, bcl-w, bcl-x, bromodeoxyuridine, CAK (cdk-activating kinase), cellular apoptosis susceptibility protein (CAS), caspase 2, caspase 8, CPP32 (caspase-3), CPP32 (caspase-3), cyclin dependent kinases, cyclin A, cyclin B1, cyclin D1, cyclin D2, cyclin D3, cyclin E, cyclin G, DNA fragmentation factor (N-terminus), Fas (CD95), Fas-associated death domain protein, Fas ligand, Fen-1, IPO-38, Mc1-1, minichromosome maintenance proteins, mismatch repair protein (MSH2), poly (ADP-Ribose) polymerase, proliferating cell nuclear antigen, p16 protein, p27 protein, p34cdc2, p57 protein (Kip2), p105 protein, Stat 1 alpha, topoisomerase I, topoisomerase II alpha, topoisomerase III alpha, topoisomerase II beta.

Neural tissue and tumor markers include alpha B crystallin, alpha-internexin, alpha synuclein, amyloid precursor protein, beta amyloid, calbindin, choline acetyltransferase, excitatory amino acid transporter 1, GAP43, glial fibrillary acidic protein, glutamate receptor 2, myelin basic protein, nerve growth factor receptor (gp75), neuroblastoma marker, neurofilament 68 kD, neurofilament 160 kD, neurofilament 200 kD, neuron specific enolase, nicotinic acetylcholine receptor alpha4, nicotinic acetylcholine receptor beta2, peripherin, protein gene product 9, S-100 protein, serotonin, SNAP-25, synapsin I, synaptophysin, tau, tryptophan hydroxylase, tyrosine hydroxylase, ubiquitin.

Cluster differentiation markers include CD1a, CD1b, CD1c, CD1d, CD1e, CD2, CD3delta, CD3epsilon, CD3gamma, CD4, CD5, CD6, CD7, CD8alpha, CD8beta, CD9, CD10, CD11a, CD11b, CD11c, CDw12, CD13, CD14, CD15, CD15s, CD16a, CD16b, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD44R, CD45, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CDw75, CDw76, CD77, CD79a, CD79b, CD80, CD81, CD82, CD83, CD84, CD85, CD86, CD87, CD88, CD89, CD90, CD91, CDw92, CDw93, CD94, CD95, CD96, CD97, CD98, CD99, CD100, CD101, CD102, CD103, CD104, CD105, CD106, CD107a, CD107b, CDw108, CD109, CD114, CD115, CD116, CD117, CDw119, CD120a, CD120b, CD121a, CDw121b, CD122, CD123, CD124, CDw125, CD126, CD127, CDw128a, CDw128b, CD130, CDw131, CD132, CD134, CD135, CDw136, CDw137, CD138, CD139, CD140a, CD140b, CD141, CD142, CD143, CD144, CDw145, CD146, CD147, CD148, CDw149, CDw150, CD151, CD152, CD153, CD154, CD155, CD156, CD157, CD158a, CD158b, CD161, CD162, CD163, CD164, CD165, CD166, and TCR-zeta.

Other cellular markers include centromere protein-F (CENP-F), giantin, involucrin, lamin A&C [XB 10], LAP-70, mucin, nuclear pore complex proteins, p180 lamellar body protein, ran, r, cathepsin D, Ps2 protein, Her2-neu, P53, S100, epithelial marker antigen (EMA), TdT, MB2, MB3, PCNA, and Ki67.

### Alternative Embodiments

In a first alternative aspect of the present disclosure is a method for quantitatively determining an estimated fixation duration of an at least partially fixed test biological specimen comprising: obtaining test spectral data from the at least partially fixed test biological specimen, wherein the test spectral data includes vibrational spectral data derived from at least a portion of the biological specimen; deriving fixation features from the obtained test spectral data using a trained fixation estimation engine; and quantitatively determining the estimated fixation duration of the at least partially fixed biological specimen based on the derived fixation features. In some embodiments, the vibrational spectral data includes mid-infrared (mid-IR) spectral data. In some embodiments, the vibrational spectral data includes Raman spectral data. In some embodiments, the system further includes operations for estimating a fixation quality using the trained fixation estimation engine.

In some embodiments, the fixation estimation engine is trained using training spectral data sets acquired from a plurality of differentially fixed training biological specimens. In some embodiments, the fixation estimation engine is trained using one or more training spectral data sets, wherein each training spectral data set includes a plurality of training vibrational spectra derived from a plurality of differentially fixed training tissue samples, and wherein each training vibrational spectrum includes class labels of a known fixation duration. In some embodiments, the class labels of known fixation duration are verified through functional IHC testing. In some embodiments, the class labels further include fixation quality annotations.

In some embodiments, each training spectral data set is derived by: (i) obtaining a training biological specimen; (ii) dividing the obtained training biological specimen into a plurality of training tissue samples; and (iii) fixing each training tissue sample of the plurality of training tissue samples for a different pre-determined amount of time. In some embodiments, the different pre-determined amount of time range from between about 0 hours to about 24 hours. In some embodiments, the different pre-determined amount of time range from between about 0 hours to about 12 hours.

The test spectral data includes an averaged vibrational spectrum derived from a plurality of normalized and corrected vibrational spectra. The plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region. In some embodiments, the acquired vibrational spectrum from each individual region is corrected by: (i) compensating each acquired vibrational spectrum for atmospheric effects to provide an atmospheric corrected vibrational spectrum; and (ii) compensating the atmospheric corrected vibrational spectrum for scattering. In some embodiments, wherein the regions are selected randomly.

In some embodiments, the trained fixation status estimation engine includes a machine learning algorithm based on dimensionality reduction. In some embodiments, dimensionality reduction includes a projection onto latent structure regression model. In some embodiments, the dimensionality reduction includes a principal component analysis and optionally discriminant analysis. In some embodiments, the trained fixation status estimation engine includes a neural network.

In some embodiments, the method further comprises assessing whether the biological specimen includes a fixation state suitable for labeling with one or more specific binding entities. In some embodiments, the method further comprises identifying at least one spectral band within the test data which is positively associated with biological specimen fixation.

In a second alternative aspect of the present disclosure is a system for determining an estimated fixation duration of an at least partially fixed test biological specimen, the system comprising: (i) one or more processors, and (ii) one or more memories coupled to the one or more processors, the one or more memories to store computer-executable instructions that, when executed by the one or more processors, cause the system to perform operations comprising: obtaining test spectral data from the test biological specimen, wherein the test spectral data includes vibrational spectral data derived from at least a portion of the biological specimen; deriving fixation features from the obtained test spectral data using a trained fixation estimation engine, wherein the fixation estimation engine is trained using training spectral data sets acquired from a plurality of differentially fixed training biological specimens and wherein the training spectral data sets include at least class labels of known fixation durations; quantitatively determining an estimated fixation duration of the at least partially fixed biological specimen based on the derived fixation features. In some embodiments, the class labels of known fixation durations used during training of the fixation estimation engine are verified through functional IHC testing. In some embodiments, the test biological specimen is unstained. In some embodiments, the best biological specimen is stained for the presence of one or more biomarkers.

In some embodiments, each training spectral data set is derived by: (i) obtaining a training biological specimen; (ii) dividing the obtained training biological specimen into a plurality of training tissue samples; and (iii) fixing each training tissue sample of the plurality of training tissue samples for a different pre-determined amount of time. In some embodiments, the training biological specimens include the same tissue type as the test biological specimen. In some embodiments, the training biological specimens include a different tissue type than the test biological specimen. In some embodiments, the trained fixation status estimation engine includes a machine learning algorithm based on dimensionality reduction. In some embodiments, the dimensionality reduction includes a projection onto latent structure regression model. In some embodiments, the dimensionality reduction includes a principal component analysis. In some embodiments, the trained fixation status estimation engine includes a neural network.

In a third alternative aspect of the present disclosure is a system for predicting a fixation state of an at least partially fixed test biological specimen, the system comprising: (i) one or more processors, and (ii) one or more memories coupled to the one or more processors, the one or more memories to store computer-executable instructions that, when executed by the one or more processors, cause the system to perform operations comprising: obtaining test spectral data from the at least partially fixed test biological specimen, wherein the test spectral data includes vibrational spectral data derived from at least a portion of the biological specimen; deriving one or more fixation features from the obtained test spectral data using a trained fixation estimation engine; and quantitatively determining an estimated fixation state of the at least partially fixed biological specimen based on the derived one or more fixation features. The fixation state is a fixation duration. In some embodiments, the method further includes assessing whether the biological specimen includes a fixation state suitable for labeling with one or more specific binding entities. In some embodiments, the method further includes identifying at least one spectral band within the test data which is positively associated with biological specimen fixation.

In some embodiments, the fixation estimation engine is trained using training spectral data sets acquired from a plurality of differentially fixed training biological specimens. In some embodiments, the training spectral data sets include class labels of known fixation durations, such as known fixation durations determined through functional IHC testing. In some embodiments, the training spectral data sets further include class labels of fixation quality.

In some embodiments, at least two training vibrational spectra are acquired from each individual training biological specimen of the plurality of the training biological specimens, and wherein the at least two sample vibrational spectra are acquired from different portions of the individual training biological specimen. In some embodiments, the at least two different portions of the individual training biological specimen are each treated with one or more fixatives for a different pre-determined amount of time. In some embodiments, the different pre-determined amounts of time range from between about 0 hours to about 24 hours. In some embodiments, the different pre-determined amounts of time range from between about 0 hours to about 12 hours. In some embodiments, at least two training vibrational spectra are each an averaged vibrational spectrum derived from a plurality of normalized and corrected training vibrational spectra.

In some embodiments, the test spectral data includes mid-IR spectral information for at least an amide I band. In some embodiments, the test spectral data comprises vibrational spectral information for wavelengths ranging from between about 3200 to about 3400 cm⁻¹, about 2800 to about 2900 cm⁻¹, about 1020 to about 1100 cm⁻¹, and/or about 1520 to about 1580 cm⁻¹. In some embodiments, the test biological specimen is unstained. In some embodiments, the test biological specimen is stained for the presence of one or more biomarkers.

In some embodiments, the trained fixation status estimation engine includes a machine learning algorithm based on dimensionality reduction. In some embodiments, the dimensionality reduction includes a projection onto latent structure regression model. In some embodiments, the dimensionality reduction includes a principal component analysis. In some embodiments, the trained fixation status estimation engine includes a neural network.

Although the present invention has been described with reference to a number of illustrative embodiments, it should be understood that numerous other modifications and embodiments can be devised by those skilled in the art that will fall within the scope of the following claims.

## Claims

1. A non-transitory computer-readable medium storing instructions for determining an estimated fixation duration of an at least partially fixed test biological specimen, comprising:
(a) obtaining test spectral data from the test biological specimen, wherein the obtained test spectral data comprises vibrational spectral data derived from at least a portion of the biological specimen, wherein the obtained test spectral data comprises an averaged vibrational spectrum derived from a plurality of normalized and corrected vibrational spectra, wherein the plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region;
(b) deriving fixation features from the obtained test spectral data using a trained fixation estimation engine, wherein the fixation estimation engine is trained using training spectral data sets acquired from a plurality of differentially fixed training biological specimens and wherein the training spectral data sets comprise at least class labels of known fixation durations; **characterized by**
(c) quantitatively determining an estimated fixation duration of the at least partially fixed biological specimen based on the derived fixation features, wherein an amount of time the at least partially fixed test biological specimen has been subjected to a fixation process is predicted.

2. The non-transitory computer-readable medium of claim 1, wherein the class labels further comprise fixation quality annotations.

3. The non-transitory computer-readable medium of claim 2, further comprising operations for estimating a fixation quality using the trained fixation estimation engine.

4. The non-transitory computer-readable medium of any one of claims 1 to 3, wherein the trained fixation estimation engine comprises a machine learning algorithm based on dimensionality reduction.

5. The non-transitory computer-readable medium of claim 4, wherein the dimensionality reduction comprises a projection onto latent structure regression model.

6. The non-transitory computer-readable medium of claim 4, wherein the dimensionality reduction comprises a principal component analysis.

7. The non-transitory computer-readable medium of any one of claims 1 to 3, wherein the trained fixation estimation engine comprises a neural network.

8. A system (200) for quantitatively determining an estimated fixation duration of an at least partially fixed test biological specimen, the system comprising: (i) one or more processors (209), and (ii) one or more memories (201) coupled to the one or more processors (209), wherein the one or more memories (201) are a non-transitory computer-readable medium according to any one of claims 1 to 7, the one or more memories (201) storing computer-executable instructions that, when executed by the one or more processors (209), cause the system (200) to perform operations comprising:
a. obtaining test spectral data from the at least partially fixed test biological specimen, wherein the obtained test spectral data comprises vibrational spectral data derived from at least a portion of the biological specimen, wherein the obtained test spectral data comprises an averaged vibrational spectrum derived from a plurality of normalized and corrected vibrational spectra, wherein the plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region;
b. deriving fixation features from the obtained test spectral data using a trained fixation estimation engine (210); and
c. quantitatively determining the estimated fixation duration of the at least partially fixed biological specimen based on the derived fixation features, wherein an amount of time the at least partially fixed test biological specimen has been subjected to a fixation process is predicted.

9. The system of claim 8, further comprising operations for estimating a fixation quality using the trained fixation estimation engine (210).

10. A method for predicting a fixation state of an at least partially fixed test biological specimen comprising:
(a) obtaining test spectral data from the at least partially fixed test biological specimen (320), wherein the obtained test spectral data comprises vibrational spectral data derived from at least a portion of the biological specimen, wherein the obtained test spectral data comprises an averaged vibrational spectrum derived from a plurality of normalized and corrected vibrational spectra, wherein the plurality of normalized and corrected vibrational spectra are obtained by: (i) identifying a plurality of spatial regions within the test biological specimen; (ii) acquiring a vibrational spectrum from each individual region of the plurality of identified regions; (iii) correcting the acquired vibrational spectrum from each individual region to provide a corrected vibrational spectrum for each individual region; and (iv) amplitude normalizing the corrected vibrational spectrum from each individual region to a pre-determined global maximum to provide an amplitude normalized vibrational spectrum for each region;
(b) deriving fixation features (340) from the obtained test spectral data using a trained fixation estimation engine, wherein the fixation estimation engine is trained using training spectral data sets acquired from a plurality of differentially fixed training biological specimens; **characterized by**
(c) quantitatively determining an estimated fixation state (350) of the at least partially fixed biological specimen based on the derived fixation features, wherein an amount of time the at least partially fixed test biological specimen has been subjected to a fixation process is predicted.

11. The method of claim 10, wherein the training spectral data sets comprise class labels of known fixation durations.

12. The method of claim 11, wherein the training spectral data sets further comprise class labels comprising annotations of known fixation quality.

13. The method of claim 12, further comprising estimating a fixation quality using the trained fixation estimation engine.

14. The method of any one of claims 10 to 13, wherein at least two training vibrational spectra are acquired from each individual training biological specimen of the plurality of the training biological specimens, and wherein the at least two sample vibrational spectra are acquired from different portions of the individual training biological specimen.

15. The method of claim 14, wherein the at least two different portions of the individual training biological specimen are each treated with fixative for a different pre-determined amount of time.

16. The method of claim 14, wherein the at least two training vibrational spectra are each an averaged vibrational spectrum derived from a plurality of normalized and corrected training vibrational spectra.

17. The method of any one of claims 10 to 16, further comprising assessing whether the biological specimen comprises a fixation state suitable for labeling with one or more specific binding entities.

18. The method of any one of claims 10 to 17, further comprising identifying at least one spectral band within the test spectral data which is positively associated with biological specimen fixation.

19. The method of any one of claims 10 to 18, wherein the trained fixation estimation engine comprises a machine learning algorithm based on dimensionality reduction, wherein the dimensionality reduction comprises a projection onto latent structure regression model or wherein the dimensionality reduction comprises a principal component analysis.

## Patentansprüche

1. Nicht flüchtiges computerlesbares Medium, das Anweisungen zum Bestimmen einer geschätzten Fixierdauer eines mindestens teilweise fixierten biologischen Testpräparats speichert, umfassend:
(a) Erhalten von Testspektraldaten von dem biologischen Testpräparat, wobei die erhaltenen Testspektraldaten Schwingungsspektraldaten umfassen, die von mindestens einem Teil des biologischen Präparats abgeleitet wurden, wobei die erhaltenen Testspektraldaten ein gemitteltes Schwingungsspektrum umfassen, das von einer Vielzahl von normierten und korrigierten Schwingungsspektren abgeleitet wurde, wobei die Vielzahl von normierten und korrigierten Schwingungsspektren wie folgt erhalten werden: (i) Identifizieren einer Vielzahl von räumlichen Regionen in dem biologischen Testpräparat; (ii) Erfassen eines Schwingungsspektrums aus jeder einzelnen Region der Vielzahl von identifizierten Regionen; (iii) Korrigieren des erfassten Schwingungsspektrums aus jeder einzelnen Region, um ein korrigiertes Schwingungsspektrum für jede einzelne Region bereitzustellen; und (iv) Amplitudennormieren des korrigierten Schwingungsspektrums aus jeder einzelnen Region auf ein vorbestimmtes globales Maximum, um ein amplitudennormiertes Schwingungsspektrum für jede Region bereitzustellen;
(b) Ableiten von Fixierungsmerkmalen von den erhaltenen Testspektraldaten unter Verwendung einer trainierten Fixierungsschätzungsmaschine, wobei die Fixierungsschätzungsmaschine unter Verwendung von Trainingsspektraldatensätzen trainiert wird, die von einer Vielzahl von unterschiedlich fixierten biologischen Trainingspräparaten erfasst wurden, und wobei die Trainingsspektraldatensätze mindestens Klassenmarkierungen mit bekannter Fixierdauer umfassen; **gekennzeichnet durch**
(c) quantitatives Bestimmen einer geschätzten Fixierdauer des mindestens teilweise fixierten biologischen Präparats basierend auf den abgeleiteten Fixierungsmerkmalen, wobei eine Zeitspanne, in der das mindestens teilweise fixierte biologische Testpräparat einem Fixierungsprozess unterzogen worden ist, vorhergesagt wird.

2. Nicht flüchtiges computerlesbares Medium nach Anspruch 1, wobei die Klassenmarkierungen ferner Fixierungsqualitätsanmerkungen umfassen.

3. Nicht flüchtiges computerlesbares Medium nach Anspruch 2, ferner umfassend Vorgänge zum Schätzen einer Fixierungsqualität unter Verwendung der trainierten Fixierungsschätzungsmaschine.

4. Nicht flüchtiges computerlesbares Medium nach einem der Ansprüche 1 bis 3, wobei die trainierte Fixierungsschätzungsmaschine einen Maschinenlernalgorithmus umfasst, der auf Dimensionalitätsreduktion basiert.

5. Nicht flüchtiges computerlesbares Medium nach Anspruch 4, wobei die Dimensionalitätsreduktion ein Projektion-auf-latente-Struktur-Regressionsmodell umfasst.

6. Nicht flüchtiges computerlesbares Medium nach Anspruch 4, wobei die Dimensionalitätsreduktion eine Hauptkomponentenanalyse umfasst.

7. Nicht flüchtiges computerlesbares Medium nach einem der Ansprüche 1 bis 3, wobei die trainierte Fixierungsschätzungsmaschine ein neurales Netzwerk umfasst.

8. System (200) zum quantitativen Bestimmen einer geschätzten Fixierdauer eines mindestens teilweise fixierten biologischen Testpräparats, wobei das System Folgendes umfasst: (i) einen oder mehrere Prozessor(en) (209) und (ii) einen oder mehrere Speicher (201), der/die an den einen oder die mehreren Prozessor(en) (209) gekoppelt ist/sind, wobei der eine oder die mehreren Speicher (201) ein nicht flüchtiges computerlesbares Medium nach einem der Ansprüche 1 bis 7 ist/sind, wobei der eine oder die mehreren Speicher (201) computerausführbare Anweisungen speichert/speichern, die bei der Ausführung von dem einen oder den mehreren Prozessor(en) (209) das System (200) veranlassen, Vorgänge auszuführen, die Folgendes umfassen:
a. Erhalten von Testspektraldaten von dem mindestens teilweise fixierten biologischen Testpräparat, wobei die erhaltenen Testspektraldaten Schwingungsspektraldaten umfassen, die von mindestens einem Teil des biologischen Präparats abgeleitet wurden, wobei die erhaltenen Testspektraldaten ein gemitteltes Schwingungsspektrum umfassen, das von einer Vielzahl von normierten und korrigierten Schwingungsspektren abgeleitet wurde, wobei die Vielzahl von normierten und korrigierten Schwingungsspektren wie folgt erhalten werden: (i) Identifizieren einer Vielzahl von räumlichen Regionen in dem biologischen Testpräparat; (ii) Erfassen eines Schwingungsspektrums aus jeder einzelnen Region der Vielzahl von identifizierten Regionen; (iii) Korrigieren des erfassten Schwingungsspektrums aus jeder einzelnen Region, um ein korrigiertes Schwingungsspektrum für jede einzelne Region bereitzustellen; und (iv) Amplitudennormieren des korrigierten Schwingungsspektrums aus jeder einzelnen Region auf ein vorbestimmtes globales Maximum, um ein amplitudennormiertes Schwingungsspektrum für jede Region bereitzustellen;
b. Ableiten von Fixierungsmerkmalen von den erhaltenen Testspektraldaten unter Verwendung einer trainierten Fixierungsschätzungsmaschine (210); und
c. quantitatives Bestimmen der geschätzten Fixierdauer des mindestens teilweise fixierten biologischen Präparats basierend auf den abgeleiteten Fixierungsmerkmalen, wobei eine Zeitspanne, in der das mindestens teilweise fixierte biologische Testpräparat einem Fixierungsprozess unterzogen worden ist, vorhergesagt wird.

9. System nach Anspruch 8, ferner umfassend Vorgänge zum Schätzen einer Fixierungsqualität unter Verwendung der trainierten Fixierungsschätzungsmaschine (210).

10. Verfahren zum Vorhersagen eines Fixierungszustands eines mindestens teilweise fixierten biologischen Testpräparats, umfassend:
(a) Erhalten von Testspektraldaten von dem mindestens teilweise fixierten biologischen Testpräparat (320), wobei die erhaltenen Testspektraldaten Schwingungsspektraldaten umfassen, die von mindestens einem Teil des biologischen Präparats abgeleitet wurden, wobei die erhaltenen Testspektraldaten ein gemitteltes Schwingungsspektrum umfassen, das von einer Vielzahl von normierten und korrigierten Schwingungsspektren abgeleitet wurde, wobei die Vielzahl von normierten und korrigierten Schwingungsspektren wie folgt erhalten werden: (i) Identifizieren einer Vielzahl von räumlichen Regionen in dem biologischen Testpräparat; (ii) Erfassen eines Schwingungsspektrums aus jeder einzelnen Region der Vielzahl von identifizierten Regionen; (iii) Korrigieren des erfassten Schwingungsspektrums aus jeder einzelnen Region, um ein korrigiertes Schwingungsspektrum für jede einzelne Region bereitzustellen; und (iv) Amplitudennormieren des korrigierten Schwingungsspektrums aus jeder einzelnen Region auf ein vorbestimmtes globales Maximum, um ein amplitudennormiertes Schwingungsspektrum für jede Region bereitzustellen;
(b) Ableiten von Fixierungsmerkmalen (340) von den erhaltenen Testspektraldaten unter Verwendung einer trainierten Fixierungsschätzungsmaschine, wobei die Fixierungsschätzungsmaschine unter Verwendung von Trainingsspektraldatensätzen trainiert wird, die von einer Vielzahl von unterschiedlich fixierten biologischen Trainingspräparaten erfasst wurden; **gekennzeichnet durch**
(c) quantitatives Bestimmen eines geschätzten Fixierzustands (350) des mindestens teilweise fixierten biologischen Präparats basierend auf den abgeleiteten Fixierungsmerkmalen, wobei eine Zeitspanne, in der das mindestens teilweise fixierte biologische Testpräparat einem Fixierungsprozess unterzogen worden ist, vorhergesagt wird.

11. Verfahren nach Anspruch 10, wobei die Trainingsspektraldatensätze Klassenmarkierungen mit bekannter Fixierdauer umfassen.

12. Verfahren nach Anspruch 11, wobei die Trainingsspektraldatensätze ferner Klassenmarkierungen umfassen, die Anmerkungen mit bekannter Fixierungsqualität umfassen.

13. Verfahren nach Anspruch 12, ferner umfassend das Schätzen einer Fixierungsqualität unter Verwendung der trainierten Fixierungsschätzungsmaschine.

14. Verfahren nach einem der Ansprüche 10 bis 13, wobei mindestens zwei Trainingsschwingungsspektren aus jedem einzelnen biologischen Trainingspräparat der Vielzahl von biologischen Trainingspräparaten erfasst werden und wobei die mindestens zwei Probenschwingungsspektren aus verschiedenen Teilen des einzelnen biologischen Trainingspräparats erfasst werden.

15. Verfahren nach Anspruch 14, wobei die mindestens zwei verschiedenen Teile des einzelnen biologischen Trainingspräparats jeweils mit Fixierungsmittel für eine andere vorbestimmte Zeitspanne behandelt werden.

16. Verfahren nach Anspruch 14, wobei die mindestens zwei Trainingsschwingungsspektren jeweils ein gemitteltes Schwingungsspektrum sind, das von einer Vielzahl von normierten und korrigierten Trainingsschwingungsspektren abgeleitet wird.

17. Verfahren nach einem der Ansprüche 10 bis 16, ferner umfassend das Beurteilen, ob das biologische Präparat einen Fixierungszustand umfasst, der für Markieren mit einer oder mehreren spezifischen Bindungseinheiten geeignet ist.

18. Verfahren nach einem der Ansprüche 10 bis 17, ferner umfassend das Identifizieren mindestens einer Spektralbande innerhalb der Testspektraldaten, die positiv mit der Fixierung eines biologischen Präparats assoziiert ist.

19. Verfahren nach einem der Ansprüche 10 bis 18, wobei die trainierte Fixierungsschätzungsmaschine einen Maschinenlernalgorithmus umfasst, der auf Dimensionalitätsreduktion basiert, wobei die Dimensionalitätsreduktion ein Projektion-auf-latente-Struktur-Regressionsmodell umfasst oder wobei die Dimensionalitätsreduktion eine Hauptkomponentenanalyse umfasst.

## Revendications

1. Support lisible par ordinateur non transitoire stockant des instructions pour déterminer une durée de fixation estimée d'un échantillon biologique de test au moins partiellement fixé, comprenant :
(a) l'obtention de données spectrales de test à partir de l'échantillon biologique de test, dans lequel les données spectrales de test obtenues comprennent des données spectrales vibrationnelles dérivées d'au moins une partie de l'échantillon biologique, dans lequel les données spectrales de test obtenues comprennent un spectre vibrationnel moyenné dérivé d'une pluralité de spectres vibrationnels normalisés et corrigés, dans lequel la pluralité de spectres vibrationnels normalisés et corrigés sont obtenus en : (i) identifiant une pluralité de régions spatiales au sein de l'échantillon biologique de test ; (ii) acquérant un spectre vibrationnel à partir de chaque région individuelle de la pluralité de régions identifiées ; (iii) corrigeant le spectre vibrationnel acquis à partir de chaque région individuelle pour fournir un spectre vibrationnel corrigé pour chaque région individuelle ; et (iv) normalisant l'amplitude du spectre vibrationnel corrigé à partir de chaque région individuelle à un maximum global prédéterminé pour fournir un spectre vibrationnel normalisé en amplitude pour chaque région ;
(b) la dérivation de caractéristiques de fixation à partir des données spectrales de test obtenues en utilisant un moteur d'estimation de fixation entraîné, dans lequel le moteur d'estimation de fixation est entraîné en utilisant des ensembles de données spectrales d'entraînement acquises à partir d'une pluralité d'échantillons biologiques d'entraînement fixés de manière différentielle et dans lequel les ensembles de données spectrales d'entraînement comprennent au moins des marqueurs de classe de durées de fixation connues ; **caractérisé par**
(c) la détermination de manière quantitative d'une durée de fixation estimée de l'échantillon biologique au moins partiellement fixé en se basant sur les caractéristiques de fixation dérivées, dans lequel une durée pendant laquelle l'échantillon biologique de test au moins partiellement fixé a été soumis à un processus de fixation est prédite.

2. Support lisible par ordinateur non transitoire selon la revendication 1, dans lequel les marqueurs de classe comprennent en outre des annotations de qualité de fixation.

3. Support lisible par ordinateur non transitoire selon la revendication 2, comprenant en outre des opérations pour estimer une qualité de fixation en utilisant le moteur d'estimation de fixation entraîné.

4. Support lisible par ordinateur non transitoire selon l'une quelconque des revendications 1 à 3, dans lequel le moteur d'estimation de fixation entraîné comprend un algorithme d'apprentissage machine basé sur la réduction de dimensionnalité.

5. Support lisible par ordinateur non transitoire selon la revendication 4, dans lequel la réduction de dimensionnalité comprend une projection sur un modèle de régression de structure latente.

6. Support lisible par ordinateur non transitoire selon la revendication 4, dans lequel la réduction de dimensionnalité comprend une analyse en composantes principales.

7. Support lisible par ordinateur non transitoire selon l'une quelconque des revendications 1 à 3, dans lequel le moteur d'estimation de fixation entraîné comprend un réseau neuronal.

8. Système (200) pour déterminer quantitativement une durée de fixation estimée d'un échantillon biologique de test au moins partiellement fixé, le système comprenant : (i) un ou plusieurs processeurs (209), et (ii) une ou plusieurs mémoires (201) couplées au ou aux processeurs (209), dans lequel la ou les mémoires (201) sont un support lisible par ordinateur non transitoire selon l'une quelconque des revendications 1 à 7, la ou les mémoires (201) stockant des instructions exécutables par ordinateur qui, lorsqu'elles sont exécutées par le ou les processeurs (209), amènent le système (200) à réaliser les opérations comprenant :
a. l'obtention de données spectrales de test à partir de l'échantillon biologique de test au moins partiellement fixé, dans lequel les données spectrales de test obtenues comprennent des données spectrales vibrationnelles dérivées d'au moins une partie de l'échantillon biologique, dans lequel les données spectrales de test obtenues comprennent un spectre vibrationnel moyenné dérivé d'une pluralité de spectres vibrationnels normalisés et corrigés, dans lequel la pluralité de spectres vibrationnels normalisés et corrigés sont obtenus en : (i) identifiant une pluralité de régions spatiales au sein de l'échantillon biologique de test ; (ii) acquérant un spectre vibrationnel à partir de chaque région individuelle de la pluralité de régions identifiées ; (iii) corrigeant le spectre vibrationnel acquis à partir de chaque région individuelle pour fournir un spectre vibrationnel corrigé pour chaque région individuelle ; et (iv) normalisant l'amplitude du spectre vibrationnel corrigé à partir de chaque région individuelle à un maximum global prédéterminé pour fournir un spectre vibrationnel normalisé en amplitude pour chaque région ;
b. la dérivation de caractéristiques de fixation à partir des données spectrales de test obtenues en utilisant un moteur d'estimation de fixation entraîné (210) ; et
c. la détermination de manière quantitative de la durée de fixation estimée de l'échantillon biologique au moins partiellement fixé en se basant sur les caractéristiques de fixation dérivées, dans lequel une durée pendant laquelle l'échantillon biologique de test au moins partiellement fixé a été soumis à un processus de fixation est prédite.

9. Système selon la revendication 8, comprenant en outre des opérations pour estimer une qualité de fixation en utilisant le moteur d'estimation de fixation entraîné (210).

10. Procédé de prédiction d'un état de fixation d'un échantillon biologique de test au moins partiellement fixé comprenant :
(a) l'obtention de données spectrales de test à partir de l'échantillon biologique de test (320) au moins partiellement fixé, dans lequel les données spectrales de test obtenues comprennent des données spectrales vibrationnelles dérivées d'au moins une partie de l'échantillon biologique, dans lequel les données spectrales de test obtenues comprennent un spectre vibrationnel moyenné dérivé d'une pluralité de spectres vibrationnels normalisés et corrigés, dans lequel la pluralité de spectres vibrationnels normalisés et corrigés sont obtenus en : (i) identifiant une pluralité de régions spatiales au sein de l'échantillon biologique de test ; (ii) acquérant un spectre vibrationnel à partir de chaque région individuelle de la pluralité de régions identifiées ; (iii) corrigeant le spectre vibrationnel acquis à partir de chaque région individuelle pour fournir un spectre vibrationnel corrigé pour chaque région individuelle ; et (iv) normalisant l'amplitude du spectre vibrationnel corrigé à partir de chaque région individuelle à un maximum global prédéterminé pour fournir un spectre vibrationnel normalisé en amplitude pour chaque région ;
(b) la dérivation de caractéristiques de fixation (340) à partir des données spectrales de test obtenues en utilisant un moteur d'estimation de fixation entraîné, dans lequel le moteur d'estimation de fixation est entraîné en utilisant des ensembles de données spectrales d'entraînement acquises à partir d'une pluralité d'échantillons biologiques d'entraînement fixés de manière différentielle ; **caractérisé par**
(c) la détermination quantitative d'un état de fixation estimé (350) de l'échantillon biologique au moins partiellement fixé en se basant sur les caractéristiques de fixation dérivées, dans lequel une durée pendant laquelle l'échantillon biologique de test au moins partiellement fixé a été soumis à un processus de fixation est prédite.

11. Procédé selon la revendication 10, dans lequel les ensembles de données spectrales d'entraînement comprennent des marqueurs de classe de durées de fixation connues.

12. Procédé selon la revendication 11, dans lequel les ensembles de données spectrales d'entraînement comprennent en outre des marqueurs de classe comprenant des annotations de qualité de fixation connue.

13. Procédé selon la revendication 12, comprenant en outre l'estimation d'une qualité de fixation en utilisant le moteur d'estimation de fixation entraîné.

14. Procédé selon l'une quelconque des revendications 10 à 13, dans lequel au moins deux spectres vibrationnels d'entraînement sont acquis à partir de chaque échantillon biologique d'entraînement individuel de la pluralité des échantillons biologiques d'entraînement, et dans lequel les au moins deux spectres vibrationnels d'échantillon sont acquis à partir de parties différentes de l'échantillon biologique d'entraînement individuel.

15. Procédé selon la revendication 14, dans lequel les au moins deux parties différentes de l'échantillon biologique d'entraînement individuel sont chacune traitées avec un fixateur pendant une durée prédéterminée différente.

16. Procédé selon la revendication 14, dans lequel les au moins deux spectres vibrationnels d'entraînement sont chacun un spectre vibrationnel moyenné dérivé d'une pluralité de spectres vibrationnels d'entraînement normalisés et corrigés.

17. Procédé selon l'une quelconque des revendications 10 à 16, comprenant en outre l'évaluation si l'échantillon biologique comprend un état de fixation approprié pour un marquage avec une ou plusieurs entités de liaison spécifiques.

18. Procédé selon l'une quelconque des revendications 10 à 17, comprenant en outre l'identification d'au moins une bande spectrale au sein des données spectrales de test qui est associée positivement à la fixation d'échantillon biologique.

19. Procédé selon l'une quelconque des revendications 10 à 18, dans lequel le moteur d'estimation de fixation entraîné comprend un algorithme d'apprentissage machine basé sur la réduction de dimensionnalité, dans lequel la réduction de dimensionnalité comprend une projection sur un modèle de régression de structure latente ou dans lequel la réduction de dimensionnalité comprend une analyse en composantes principales.
